(19) Europäisches Patentamt
European Patent Office
Office européen des brevets

(11) EP 2 058 315 A1

(12) EUROPEAN PATENT APPLICATION

(43) Date of publication:
**13.05.2009 Bulletin 2009/20**

(51) Int Cl.:
***C07D 491/04*** *(2006.01)*

(21) Application number: **08167597.7**

(22) Date of filing: **27.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 EP 07119643**

(71) Applicant: **Nerviano Medical Sciences S.r.l.**
**20014 Nerviano (MI) (IT)**

(72) Inventors:
- **Fancelli, Daniele**
  **20144, Milan (IT)**
- **Pulici, Maurizio**
  **20040, Caponago (MI) (IT)**
- **Cervi, Giovanni**
  **22100, Como (IT)**
- **Quartieri, Francesca**
  **28041, Arona (NO) (IT)**
- **Vianello, Paola**
  **20149, Milan (IT)**

(54) **1H-Furo[3,2-C]Pyrazoles active as aurora kinase inhibitors**

(57) Furo[3,2-c]pyrazole derivatives of formula (I) and pharmaceutically acceptable salts thereof, as defined in the specification, process for their preparation and pharmaceutical compositions comprising them are disclosed; the compounds of the invention can be useful, in therapy, in the treatment of disorder or conditions associated with an altered Aurora kinase activity, such as cancer.

EP 2 058 315 A1

Printed by Jouve, 75001 PARIS (FR)

## Description

## BACKGROUND OF THE INVENTION

### Field of the Invention

The present invention relates to furo-pyrazole derivatives, to a process for their preparation, to pharmaceutical compositions comprising them, and to their use as therapeutic agents, particularly in the treatment of cancer and cell proliferation disorders.

### Discussion of the Background

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

PKs are also implicated in inflammatory conditions and in the multiplication of viruses and parasites. PKs can also play a major role in the pathogenesis and development of neurodegenerative disorders.

For a general reference to PKs malfunctioning or disregulation see, for instance, Current Opinion in Chemical Biology 1999, 3, 459 - 465.

Among the several protein kinases known in the art as being implicated in the growth of cancer cells are Aurora kinases, in particular, Aurora-2.

Aurora-2 was found to be over-expressed in a number of different tumor types. Its gene locus maps at 20q13, a chromosomal region frequently amplified in many cancers, including breast [Cancer Res. 1999, 59(9), 2041-4] and colon. 20q13 amplification correlates with poor prognosis in patients with node-negative breast cancer and increased Aurora-2 expression is indicative of poor prognosis and decreased survival time in bladder cancer patients [J. Natl. Cancer Inst., 2002, 94(17), 1320-9]. For a general reference to Aurora-2 role in the abnormal centrosome function in cancer see also Molecular Cancer Therapeutics, 2003, 2, 589 - 595.

## SUMMARY OF THE INVENTION

It is an object of the invention to provide compounds, which are useful in therapy as agents against a host of diseases caused by and/or associated to a disregulated protein kinase activity and, more particularly, Aurora kinases activity.

It is another object to provide compounds which are endowed with protein kinase inhibiting activity and, more particularly, Aurora kinases inhibiting activity.

The present inventors have now found that some furo-pyrazole compounds, and derivatives thereof, are endowed with protein kinase inhibiting activity, e.g. Aurora kinases inhibiting activity.

The present invention provides a compound of formula (I)

(I)

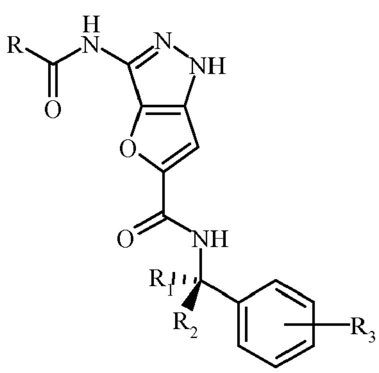

or a pharmaceutically acceptable salt of solvate thereof, wherein:

**R** is heteroaryl or phenyl group or a mono- or di-substituted heteroaryl or phenyl group in which the substituent(s)

is(are) at position(s) meta or para to the CONH linker;

**$R_1$ and $R_2$,** which can be the same or different, is a straight or branched ($C_1$-$C_3$) alkyl or, taken together with the carbon atom to which they are bonded, **$R_1$** and **$R_2$** can form a ($C_3$-$C_6$) cycloalkyl group; and

**$R_3$** is selected from the group consisting of hydrogen, halogen, hydroxy, cyano, straight or branched ($C_1$-$C_3$) alkyl and ($C_1$-$C_3$) alkoxy.

The present invention also includes methods of synthesizing the furo-pyrazole compounds of formula (I) and the pharmaceutically acceptable salts, as well as the pharmaceutical compositions comprising them.

Another aspect of the invention relates to a pharmaceutical composition comprising a therapeutically effective amount of the compound of formula (I) or a pharmaceutically acceptable salt thereof, and at least one pharmaceutically acceptable excipient, carrier and/or diluent.

Another aspect of the invention relates to a method for treating a disorder or condition caused by or associated with an altered Aurora kinase activity, comprising administering to a mammal in need of said treatment a compound of formula (I) or a pharmaceutically effective amount thereof.

A more complete appreciation of the invention and many of the attendant advantages thereof will be readily obtained as the same becomes better understood by reference to the following detailed description.

## DETAILED DESCRIPTION OF THE INVENTION

The compounds of formula (I) of the invention have tetrasubstituted carbon atoms and can therefore exist as individual optical isomers, as racemic mixtures or as any other mixture comprising a majority of one of the two optical isomers, which are all to be intended as within the scope of the present invention.

Likewise, the use as an antitumor agent of all the possible isomers and their admixtures and of both the metabolites and the pharmaceutically acceptable bio-precursors (otherwise referred to as pro-drugs) of the compounds of formula (I) are also within the scope of the present invention. Prodrugs are any covalently bonded compounds, which release the active parent drug, according to formula (I), in vivo.

In cases when compounds can exist in tautomeric forms, each form is contemplated as being included within this invention whether existing in equilibrium or predominantly in one form.

As such, unless otherwise provided, when only one of the following tautomeric forms of formula (Ia) or (Ib) is indicated, the remaining one has still to be intended as comprised within the scope of the invention:

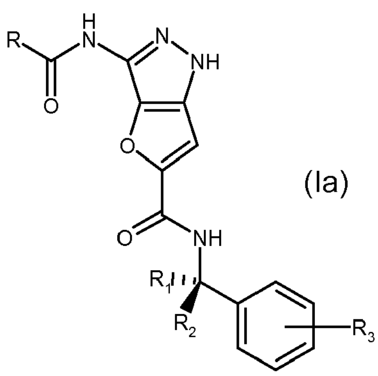

(Ia)

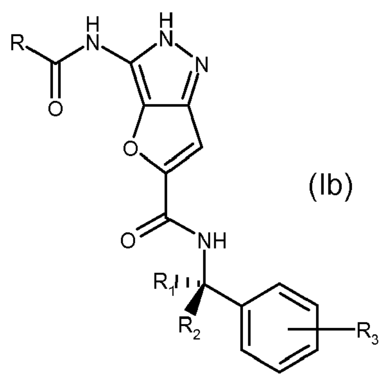

(Ib)

In the present description, unless otherwise specified, with the term "aryl" we intend any aromatic carbocyclic ring system of one or two ring moieties, either fused or linked to each other through a single bond, for instance including phenyl, phenyl, α- or β-naphthyl or biphenyl groups.

The term "heteroaryl" means any aromatic heterocyclic ring which can comprise an optionally benzocondensed 5 or 6 membered heterocycle with from 1 to 3 heteroatoms selected among N, O or S.

Non limiting examples of heteroaryl groups according to the invention can thus include pyridyl, pyrazinyl, pyrimidinyl, pyridazinyl, indolyl, imidazolyl, thiazolyl, isothiazolyl, pyrrolyl, phenyl-pyrrolyl, furyl, phenyl-furyl, oxazolyl, isoxazolyl, pyrazolyl, thienyl, benzothienyl, isoindolinyl, benzoimidazolyl, quinolinyl, isoquinolinyl, 1,2,3-triazolyl, 1-phenyl-1,2,3-triazolyl, and the like. The term "straight or branched ($C_1$-$C_3$) alkyl" or "($C_1$-$C_3$) alkoxy" we intend any of the groups such as methyl, ethyl, n-propyl, isopropyl, methoxy, ethoxy, n-propoxy and isopropoxy.

The term "halogen," "halo" or "halogen atom" means fluorine, chlorine, bromine or iodine.

The term "($C_3$-$C_6$) cycloalkyl" means cyclopropyl, cyclobutyl, cyclopentyl or cyclohexyl. Cycloalkyl groups can be formed when $R_1$ and $R_2$ are taken together, with the carbon atom to which they are attached, to form cyclic spiro compounds. For example, when $R_1$ and $R_2$, together form a cyclopentyl group, derivatives having the following general formula are herewith considered:

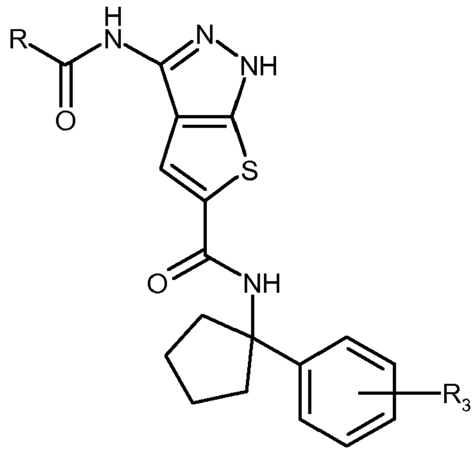

Aryl or heteroaryl groups can be unsubstited or substituted in any of their free positions by one or more groups, for instance, 1 to 6 groups, wherein each group is selected from the group consisting of halogen, nitro, oxo groups (=O), carboxy, cyano, alkyl, polyfluorinated alkyl, alkenyl, alkynyl, cycloalkyl, cycloalkenyl, aryl, heterocyclyl, alkyl-heterocyclyl, heterocyclyl-alkyl, amino groups and derivatives thereof such as, for instance, alkylamino, dialkylamino, arylamino, diarylamino, ureido, alkylureido or arylureido; carbonylamino groups and derivatives thereof, such as, for instance, formylamino, alkylcarbonylamino, alkenylcarbonylamino, arylcarbonylamino, alkoxycarbonylamino; hydroxy groups and derivatives thereof such as, for instance, alkoxy, polyfluorinated alkoxy, aryloxy, heterocylyloxy, alkylcarbonyloxy, arylcarbonyloxy, cycloalkenyloxy or alkylideneaminoxy; carbonyl groups and derivatives thereof such as, for instance, alkylcarbonyl, arylcarbonyl, alkoxycarbonyl, aryloxycarbonyl, cycloalkyloxycarbonyl, aminocarbonyl, alkylaminocarbonyl, dialkylaminocarbonyl, and sulfurated derivatives such as, for instance, alkylthio, arylthio, alkylsulfonyl, arylsulfonyl, alkylsulfinyl, arylsulfinyl, arylsulfonyloxy, aminosulfonyl, alkylaminosulfonyl or dialkylaminosulfonyl.

In their turn, whenever appropriate, each of the above substituents can be further substituted by one or more of the aforementioned groups.

The term "alkyl" means an aliphatic hydrocarbon group, which can be straight or branched.

Alkyl groups include ($C_1$-$C_6$) alkyl groups, ($C_1$-$C_4$) alkyl groups and ($C_1$-$C_3$) alkyl groups which are alkyl group having, respectively, 1-6, 1-4 and 1-3 carbon atoms. Branched means that one or more alkyl groups, such as methyl, ethyl or propyl, are attached to a linear alkyl chain. Nonlimiting examples of alkyl groups include methyl, ethyl, n-propyl, isopropyl, n-butyl, isobutyl, tert-butyl, sec-butyl, n-pentyl, n-hexyl, and the like. The alkyl group can be substituted by one or more substituents which can each be independently selected from the group consisting of halo, alkyl, aryl, cycloalkyl, cyano, hydroxy, alkoxy, alkylthio, amino, -NH(alkyl), -NH(cycloalkyl), $-N(alkyl)_2$, carboxy and -C(O)O-alkyl. Non-limiting examples of suitable alkyl groups include methyl, ethyl, n-propyl, isopropyl, n-butyl, and t-butyl, isobutyl, sec-butyl, n-pentyl, n-hexyl, and the like.

The term "alkenyl" means an aliphatic hydrocarbon group containing at least one carbon-carbon double bond and which can be straight or branched. Alkenyl groups include ($C_2$-$C_6$) alkenyl groups, which is an alkenyl group having 2-6 carbon atoms, and ($C_2$-$C_3$)alkenyl groups, which have 2-3 carbon atoms, respectively. The term "substituted alkenyl" means that the alkenyl group can be substituted by one or more substituents which can be the same or different, each substituent being independently selected from the group consisting of halo, alkyl, aryl, cycloalkyl, cyano, and alkoxy. Non-limiting examples of suitable alkenyl groups include ethenyl, propenyl, and n-butenyl.

The term "alkynyl" means an aliphatic hydrocarbon group containing at least one carbon-carbon triple bond and which can be straight or branched. Alkynyl groups include ($C_2$-$C_6$) alkynyl groups, which is an alkynyl group having 2-6 carbon atoms, and ($C_2$-$C_3$) alkynyl groups, which have 2-3 carbon atoms, respectively. Branched means that one or more lower alkyl groups such as methyl, ethyl or propyl, are attached to a linear alkynyl chain. Non-limiting examples of suitable alkynyl groups include ethynyl, propynyl, and 2-butynyl. The term "substituted alkynyl" means that the alkynyl group can be substituted by one or more substituents each being independently selected from the group consisting of alkyl, aryl and cycloalkyl.

The term "amino" means an $-NH_2$ group whilst the term arylamino comprises any group -NH-aryl, wherein aryl

is as defined below.

The term "alkoxy," as used herein, means an oxygen atom bonded to a hydrocarbon chain, such as an alkyl or alkenyl group (e.g., -O-alkyl or -O-alkenyl). Representative alkoxy groups include methoxy, ethoxy, and isopropoxy groups.

Prodrugs and solvates of the compounds of the invention are also contemplated herein. The term "prodrug", as employed herein, denotes a compound that is a drug precursor, which, upon administration to a subject, undergoes chemical conversion by metabolic or chemical processes to yield a compound of formula (I) or a salt and/or solvate thereof. A discussion of prodrugs is provided in T. Higuchi and V. Stella, Pro-drugs as Novel Delivery Systems (1987) Volume 14 of the A.C.S. Symposium Series, and in Bioreversible Carriers in Drug Design, (1987) Edward B. Roche, ed., American Pharmaceutical Association and Pergamon Press, both of which are incorporated herein by reference thereto. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates.

Non-limiting examples of suitable solvates include ethanolates, methanolates, and the like. "Hydrate" is a solvate wherein the solvent molecule is $H_2O$.

When any variable (e.g., aryl, alkyl, etc.) occurs more than one time in any constituent or in Formula (I), its definition on each occurrence is independent of its definition at every other occurrence. Also, combinations of substituents and/or variables are permissible only if such combinations result in stable compounds.With the term alkenyl or alkynyl group we intend, unless otherwise provided, any unsaturated straight or branched ($C_2$-$C_6$) alkenyl or alkynyl group such as, for instance, vinyl, allyl, 1-propenyl, isopropenyl, 1-, 2- or 3-butenyl, pentenyl, hexenyl, ethynyl, 1- or 2-propynyl, butynyl, pentynyl, hexynyl, and the like.

The term polyfluorinated alkyl or alkoxy means any straight or branched $C_1$-$C_6$ alkyl or alkoxy group as above defined, wherein more than one hydrogen atom is replaced by fluorine atoms such as, for instance, trifluoromethyl, trifluoromethoxy, 2,2,2-trifluoroethyl, 2,2,2-trifluoroethoxy, 1,2-difluoroethyl, 1,1,1,3,3,3-hexafluoropropyl-2-yl, and the like.

The term heterocycle, heterocyclyl or heterocyclic means an optionally benzocondensed 4 to 7 membered heterocycle, hence encompassing aromatic heterocyclic groups also known as heteroaryl groups, either saturated or partially unsaturated, with from 1 to 3 heteroatoms selected among N, O and S. Examples of these 4 or 7 membered heterocyclic groups include 1,3-dioxolane, pyran, pyrrolidine, pyrroline, imidazoline, imidazolidine, pyrazolidine, pyrazoline, piperidine, piperazine, morpholine, tetrahydrofuran, hexamethyleneimine, 1,4-hexahydrodiazepine, azetidine, and the like.

The term cycloalkenyl means any of the aforementioned ($C_3$-$C_6$) cycloalkyl groups further comprising a double bond such as, for instance, 2-cyclopenten-1-yl, 3-cyclopenten-1-yl, 1-cyclohexen-1-yl, 2-cyclohexen-1-yl, 3-cyclohexen-1-yl, and the like.

From all of the above, it is clear to the skilled artisian that any group which name has been identified as a composite name, such as cycloalkylalkyl, arylalkyl, heterocyclylalkyl, alkylthio, aryloxy, arylalkyloxy, alkylcarbonyloxy and the like, has to be intended as conventionally construed from the parts to which they derive. So far, as an example, the term alkoxy-heterocyclyl-alkyl stands for a straight or branched alkyl group substituted by a heterocycle further substituted by alkoxy, wherein alkyl, heterocycle and alkoxy are as above defined. Likewise, the term alkyl-heterocyclyloxy stands for a heterocyclyloxy group further substituted by alkyl.

The term "pharmaceutically acceptable salts" embraces salts commonly used to form alkali metal salts and to form addition salts of free acids or free bases. The nature of the salt is not critical, provided that it is pharmaceutically acceptable. Suitable pharmaceutically acceptable acid addition salts of the compounds of the present invention can be prepared from an inorganic or organic acid. Examples of such inorganic acids include hydrochloric, hydrobromic, hydroiodic, nitric, carbonic, sulfuric, and phosphoric acid. Appropriate organic acids can be selected from aliphatic, cycloaliphatic, aromatic, arylaliphatic, heterocyclic, carboxylic and sulfonic classes of organic acids, examples of which are formic, acetic, trifluoroacetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, stearic, cyclohexylaminosulfonic, algenic, hydroxybutyric, galactaric and galacturonic acid. Suitable pharmaceutically acceptable base addition salts of the compounds of the present invention include metallic salts made from aluminum, calcium, lithium, magnesium, potassium, sodium and zinc or organic salts made from N, N'-dibenzylethylenediamine, chloroprocaine, choline, diethanolamine, ethylenediamine, meglumine (N-methyl-glucamine) and procaine. All of these salts can be prepared by conventional means from the corresponding compounds of the present invention, for instance by reacting them with the appropriate acid or base.

A preferred class of compounds of the invention is represented by the derivatives of formula (I) wherein R is selected from the group consisting of thienyl, furyl, pyrrolyl and phenyl.

In another embodiment, R is selected from the group consisting of thienyl, furyl, pyrrolyl, N-methyl-pyrrolyl, unsubstituted phenyl, phenyl substituted with 1-5 halogen atoms, heterocycles, heterocylyloxy or heterocylylalkyl groups.

Even more preferably, within the above class of compounds of formula (I), R is selected from the group consisting of 2-thienyl, 2-furyl, 1-methyl-pyrrolyl-2-yl, phenyl, 4-fluorophenyl, 4-(1-methyl-piperidin-4-yloxy)phenyl, 4-(1-methyl-piperazinyl-4-yl)phenyl, 4-(1-methyl-piperazinyl-4yl-methyl)phenyl and 4-(morpholino-4-yl)phenyl. Another preferred class of compounds of the invention is represented by the derivatives of formula (I) wherein $R_1$ and $R_2$, together with the carbon atom to which they are attached, form a $C_3$-$C_6$ cycloalkyl such as cyclopropyl or cyclopentyl. Another preferred class of compounds of the invention is represented by the derivatives of formula (I) wherein R, $R_1$ and $R_2$ are as set forth above and $R_3$ is selected form the group consisting of hydrogen, fluorine, chlorine, hydroxy, methoxy and cyano. For a reference to any specific compound of formula (I) of the invention, optionally in the form of a pharmaceutically acceptable salt, see the following experimental section. As formerly indicated, a further object of the present invention is represented by a process for preparing the compounds of formula (I), and the pharmaceutically acceptable salts or solvates thereof, which process comprises:

a) reacting a bi-cyclic compound of formula (II)

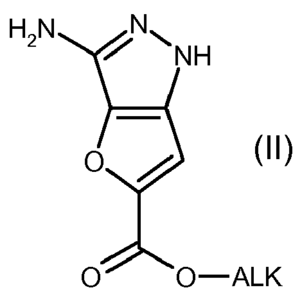

(II)

wherein ALK is a $C_1$-$C_4$ alkyl group, with a suitable pyrazole nitrogen atom protecting agent, such as ethyl chloroformate,

b) acylating the resultant compound of formula (III)

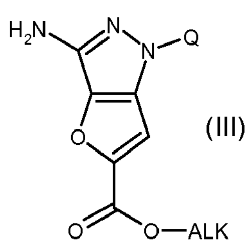

(III)

wherein ALK is as defined above and Q represents any suitable pyrazole protecting group, such as, for example, ethyloxycarbonyl, with a compound of formula (IV)

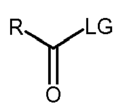

(IV)

wherein R is as defined in formula (I), and LG represents a suitable leaving group;

c) hydrolysing the alkyl ester group and removing the protective group Q from the compound of formula (V)

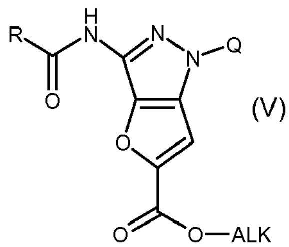

(V)

wherein ALK, R and Q are as defined above; and
d) reacting the resultant compound of formula (VI)

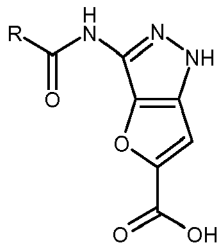

(VI)

wherein R is as defined above, with a compound of formula (VII)

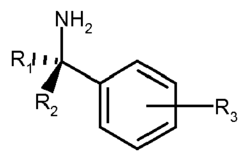

(VII)

wherein $R_1$, $R_2$ and $R_3$ are as defined in Formula (I), in the presence of a suitable condensing agent, as to obtain the compound of formula (I) and, whenever desired, converting the compound of formula (I) into a pharmaceutically acceptable salt or converting the salt thereof into the free compound of formula (I). The above process is an analogy process, which can be carried out according to methods known in the art.
From the above, it is clear to the person skilled in the art that if a compound of formula (I), prepared according to the above process, is obtained as an admixture of isomers, their separation into the single isomers of formula (I), carried out according to conventional techniques, is still within the scope of the present invention.
Whenever desired, any of the resultant compound of formula (I), as defined above, can be converted into a different compound of formula (I) by well known reactions or converted into a pharmaceutically acceptable salt.
It is to be noted that a compound of formula (I), (II) and (VI) as above defined can be in any one of its tautomeric forms a or b:

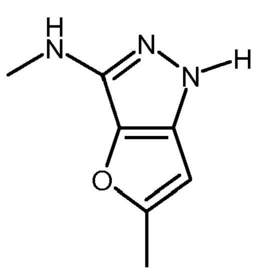

*a*

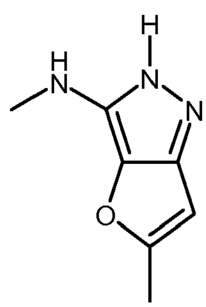

*b*

According to step (a) of the process, the furo-pyrazole derivative of formula (II) is protected, according to well-known methods, at the pyrazole nitrogen atom. As an example, the above protection can occur with an alkyl chlorocarbonate,

in a suitable solvent such as tetrahydrofuran, dichloromethane, chloroform, acetonitrile, toluene or mixtures thereof, at a temperature ranging from about -5°C to about 35°C and for a time varying from about 30 minutes to about 72 hours, in the presence of an opportune proton scavenger such as triethylamine or diisopropylethylamine. According to step (b) of the process, the compound of formula (III) is then reacted with any suitable acylating agent of formula (IV) so as to yield the compound of formula (V), by working according to methods well known in the art for the preparation of carboxamido derivatives. Typically, within the compound of formula (IV), LG represents a halogen atom and, even more preferably, a bromine or chlorine atom.

The reaction is carried out in a suitable solvent such as, for instance, tetrahydrofuran, dimethylformamide, dichloromethane, chloroform, acetonitrile, toluene or mixtures thereof, at a temperature ranging from about -10°C to reflux and for a time varying from about 30 minutes to about 96 hours, in the presence of an opportune proton scavenger such as triethylamine, N,N-diisopropylethylamine or pyridine.

According to step (c) of the process, the carboxyester function of the compound of formula (V) is hydrolyzed so as to yield the corresponding carboxy group and, at the same time, protective group Q is removed. The reaction is carried out under alkaline conditions, preferably by treatment with aqueous sodium or potassium hydroxide in the presence of a suitable co-solvent such as methanol or ethanol, by operating at a temperature ranging from room temperature to the reflux temperature of the mixture and for a suitable time, for instance up to 72 hours.

According to step (d) of the process, the compound of formula (VI) is then reacted with a suitable amino derivative of formula (VII) resulting in the corresponding compound of formula (I). From the above it is clear to the skilled person that this reaction can be accomplished in a variety of ways and operative conditions, which are widely known in the art for the preparation of carboxamides. As an example, the reaction between the compounds of formulae (VI) and (VII) can be carried out in the presence of a coupling agent such as, for instance, 2-(1H-benzotriazol-1-yl)-1,1,3,3-tetramethyluronium tetrafluoroborate (TBTU), 1,3-dicyclohexylcarbodiimide, 1,3-diisopropylcarbodiimide, 1-(3-dimethylaminopropyl)-3-ethylcarbodiimide, N-cyclohexylcarbodiimide-N'-propyloxymethyl polystyrene or N-cyclohexylcarbodiimide-N'-methyl polystyrene, in a suitable solvent such as, for instance, dichloromethane, chloroform, tetrahydrofuran, diethyl ether, 1,4-dioxane, acetonitrile, toluene, or N,N-dimethylformamide at a temperature ranging from about -10°C to reflux and for a suitable time, for instance from about 30 minutes to about 96 hours. The said reaction is optionally carried out in the presence of a suitable catalyst, for instance 4-dimethylaminopyridine, or in the presence of a further coupling reagent such as N-hydroxybenzotriazole. Alternatively, this same reaction can be also carried out, for example, through a mixed anhydride method, by using an alkyl chloroformate such as ethyl, iso-butyl, or iso-propyl chloroformate, in the presence of a tertiary base such as triethylamine, N,N-diisopropylethylamine or pyridine, in a suitable solvent such as, for instance, toluene, dichloromethane, chloroform, tetrahydrofuran, acetonitrile, diethyl ether, 1,4-dioxane, or N,N-dimethylformamide, at a temperature ranging from about -30°C to room temperature. All of the compounds of formula (II), (IV) and (VII) are known or can be obtained according to known methods.

For a reference to the preparation of the compounds of formula (II) see WO 04/007504, as well as the following experimental section.

## PHARMACOLOGY

The compounds of formula (I) are active as protein kinase inhibitors, more particularly as Aurora kinases inhibitors, and are therefore useful, for instance, to restrict the unregulated proliferation of tumor cells.

In therapy, they can be used in the treatment of various tumors, such as those formerly reported, as well as in the treatment of other cell proliferative disorders such as psoriasis, vascular smooth cell proliferation associated with atherosclerosis and post-surgical stenosis and restenosis.

The inhibiting activity and the potency of selected compounds is determined through a method of assay based on the use of the SPA technology (Amersham Pharmacia Biotech).

The assay consists of the transfer of radioactivity labelled phosphate moiety by the kinase to a biotinylated substrate. The resulting 33P-labelled biotinylated product is allowed to bind to streptavidin-coated SPA beads (biotin capacity 130 pmol/mg), and light emitted was measured in a scintillation counter.

### Inhibition assay of Aurora-2 activity

Kinase reaction: 8 μM biotinylated peptide (4 repeats of LRRWSLG, commercially available from Sigma, catalogue no. L-3523), 10 μM ATP (0.5 uCi $P^{33}\gamma$-ATP), 7.5 ng Aurora 2, inhibitor in a final volume of 30 μl buffer (HEPES 50 mM pH 7.0, $MgCl_2$ 10 mM, 1 mM DTT, 0.2 mg/mL BSA, 3 μM orthovanadate) were added to each well of a 96 U bottom well plate. After 60 minutes at room temperature incubation, the reaction was stopped and biotinylated peptide captured by adding 100 μl of bead suspension.

**Stratification**: 100 μl of CsCl 5 M were added to each well and stood for 4 hours before radioactivity was

counted in the Top-Count instrument.

**$IC_{50}$ determination**: inhibitors were tested at different concentrations ranging from 0.0015 to 10 μM. Experimental data were analyzed by the computer program GraphPad Prizm using the four parameter logistic equation: y = bottom+(top-bottom)/(1+10^((logIC50-x)*slope)) where x is the logarithm of the inhibitor concentration, y is the response; y starts at bottom and goes to top with a sigmoid shape. **Ki calculation**: **Experimental method**: Reaction was carried out in buffer (10 mM Tris, pH 7.5, 10 mM $MgCl_2$, 0.2 mg/mL BSA, 7.5 mM DTT) containing 3.7 nM enzyme, histone and ATP (constant ratio of cold/labeled ATP 1/3000). The reaction was stopped with EDTA and the substrate captured on phosphomembrane (Multiscreen 96 well plates from Millipore). After extensive washing, the multiscreen plates were read on a top counter. Control (time zero) for each ATP and histone concentrations was measured.

**Experimental design**: Reaction velocities were measured at four ATP, substrate (histone) and inhibitor concentrations. An 80-point concentration matrix was designed around the respective ATP and substrate Km values, and the inhibitor $IC_{50}$ values (0.3, 1, 3, 9 fold the Km or $IC_{50}$ values). A preliminary time course experiment in the absence of inhibitor and at the different ATP and substrate concentrations allows the selection of a single endpoint time (10 min) in the linear range of the reaction for the Ki determination experiment.

**Kinetic parameter estimates**: Kinetic parameters were estimated by simultaneous nonlinear least-square regression using [Eq.1] (competitive inhibitor respect to ATP, random mechanism) using the complete data set (80 points):

$$v = \frac{Vm \bullet A \bullet B}{\alpha \bullet Ka \bullet Kb + \alpha \bullet Ka \bullet B + a \bullet Kb \bullet A + A \bullet B + \alpha \bullet \frac{Ka}{Ki} \bullet I \bullet (Kb + \frac{B}{\beta})} \quad \text{[Eq.1]}$$

where A=[ATP], B=[Substrate], I=[inhibitor], Vm= maximum velocity, Ka, Kb, Ki are dissociation constants of ATP, substrate and inhibitor respectively. α and β the cooperativity factor between substrate and ATP binding and substrate and inhibitor binding respectively. The compounds of the invention were further tested, in vitro, to assess the anti-proliferative effect onto cell cultures.

## In vitro cell proliferation assay

The human colon cancer cell line HCT-116 was seeded at 5000 cells/$cm^2$ in 24 wells plate (Costar) using F12 medium (Gibco) supplemented with 10% FCS (EuroClone, Italy) 2 mM L-glutamine and 1% penicillin/streptomycin and maintained at 37°C, 5% $CO_2$ and 96% relative humidity. The following day, plates were treated in duplicates with 5ul of an appropriate dilution of compounds starting from a 10 mM stock in DMSO. Two untreated control wells were included in each plate. After 72 hours of treatment, medium was withdrawn and cells detached from each well using 0.5 mL of 0.05% (w/v) Trypsin, 0,02% (w/v) EDTA (Gibco). Samples were diluted with 9.5 mL of Isoton (Coulter) and counted using a Multisizer 3 cell counter (Beckman Coulter). Data were evaluated as percent of the control wells: % of CTR = (Treated - Blank)/(Control - Blank).

$IC_{50}$ values were calculated by LSW/Data Analysis using Microsoft Excel sigmoidal curve fitting. Given the above assays, the compounds of formula (I) of the invention resulted to possess a remarkable protein kinase inhibitory activity, e.g. Aurora-2 inhibitory activity. See, as an example, the following Table 1 reporting the experimental data of some representative compounds of the invention being tested as Aurora-2 kinase inhibitors (IC50 nM) and for their cell antiproliferative effect (IC50 nM), namely:

Compound (1) is 3-[(Thiophene-2-carbonyl)-amino]-1H-furo[3,2-c]pyrazole-5-carboxylic acid (1-methyl-1-phenyl-ethyl)-amide;

Compound (2) is 3-(4-Morpholin-4-ylmethyl-benzoylamino)-1H-furo[3,2-c]pyrazole-5-carboxylic acid[1-(2-fluoro-phenyl)-1-methyl-ethyl]-amide

Compound (3) is 3-[4-(4-Methyl-piperazin-1-yl)-benzoylamino]-1H-furo[3,2-c]pyrazole-5-carboxylic acid (1-phenyl-cyclopropyl)-amide

**Table 1**

| Compound | Aurora-2 inhibition $IC_{50}$ (nM) | Cell Antiproliferation (HCT-116) $IC_{50}$(nM) |
|---|---|---|
| 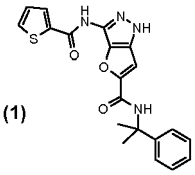 (1) | 3 | 10 |
| 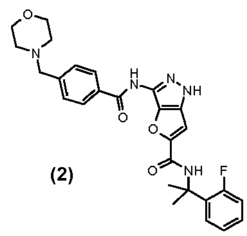 (2) | 1 | 2 |
| 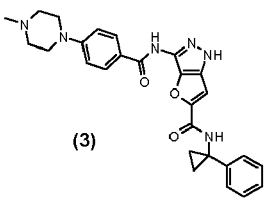 (3) | 4 | 4 |

The Aurora-2 inhibitory activity $IC_{50}$ for compounds 1-3 ranges from about 1 nM to about 4 nM, and the cell antiproliferation $IC_{50}$ ranges from about 4 nM to about 10 nM. These compounds are particularly advantageous, in therapy, against proliferative disorders associated with an altered kinase activity, in particular altered Aurora-2 kinase activity.

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

If formulated as a fixed dose, such combination products described above employ the compounds of this invention within the dosage range described below and the other pharmaceutically active agent within the approved dosage range.

Compounds of formula (I) can be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, and conditions of the patient and administration route.

For example, a suitable dosage adopted for oral administration of a compound of formula (I) can range from about 10 to about 500 mg per dose, from 1 to 5 times daily.

The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a therapeutically effective amount of the compound of formula (I) or a pharmaceutically acceptable salt thereof in association with at least one pharmaceutically acceptable excipient, which can be a carrier or a diluent.

Another object of the present invention is a method for treating a disorder or condition caused by or associated with an altered Aurora kinase activity comprising administering to a mammal in need of said treatment a compound of formula (I) or a pharmaceutically acceptable salt thereof, as defined above.

Preferably, the treated disorder or condition is selected from the group consisting of cancer, cell proliferative

disorders, viral infections, retinopathies, atherosclerosis and conditions involving vascular smooth muscle proliferation or neointimal formation, graft vessel disease, acromegaly and disorders secondary to acromegaly as well as other hypertrophic conditions in which Aurora kinase signalling is implicated, and metabolic disorders in which elevated Aurora kinase activity is implicated.

More preferably, the treated cancer can be selected from the group consisting of bladder cancer, breast cancer, colon cancer, kidney cancer, liver cancer, lung cancer, including small cell lung cancer, esophagus cancer, gall-bladder cancer, ovarian cancer, pancreatic cancer, stomach cancer, cervical cancer, thyroid cancer, prostate cancer, and skin cancer, including squamous cell carcinoma, hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma, Burkitt's lymphoma, hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

According to a further embodiment of the invention, the treated cell proliferative disorder can be selected from the group consisting of benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

A further object of the present invention is a compound of formula (I) or a pharmaceutically acceptable salt thereof, as defined in claim 1, for use as a medicament.

Another object of the present invention is the use of a compound of formula (I) or a pharmaceutically acceptable salt thereof, as defined above, in the manufacture of a medicament for treating a disorder or condition caused by or associated with an altered Aurora kinase activity.

A further object is the use of a compound of formula (I) or a pharmaceutically acceptable salt thereof, as defined above, in the manufacture of a medicament with antitumor activity.

Still a further object is a compound of formula (I) or a pharmaceutically acceptable salt thereof, as defined above, for use in a method for treating a disease caused by or associated with an altered Aurora kinase activity.

The pharmaceutical compositions containing the compounds of the invention are usually prepared following conventional methods and are administered in a suitable pharmaceutical form.

For example, the solid oral forms can contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations can be manufactured in a known manner, for example, by means of mixing, granulating, tabletting, sugar-coating, or film-coating processes.

The liquid dispersions for oral administration can be, e.g., syrups, emulsions and suspensions. As an example the syrups can contain, as a carrier, saccharose or saccharose with glycerine and/or mannitol and sorbitol.

The suspensions and the emulsions can contain, as examples of carriers, natural gum, agar, sodium alginate, pectin, methylcellulose, carboxymethylcellulose, or polyvinyl alcohol.

The suspension or solutions for intramuscular injections can contain, together with the active compound, a pharmaceutically acceptable carrier, e.g., sterile water, olive oil, ethyl oleate, glycols, e.g., propylene glycol and, if desired, a suitable amount of lidocaine hydrochloride.

The solutions for intravenous injections or infusions can contain, as a carrier, sterile water or preferably they can be in the form of sterile, aqueous, isotonic, saline solutions or they can contain propylene glycol as a carrier.

The suppositories can contain, together with the active compound, a pharmaceutically acceptable carrier, e.g., cocoa butter, polyethylene glycol, a polyoxyethylene sorbitan fatty acid ester surfactant or lecithin.

With the aim to better illustrate the present invention, without posing any limitation to it, the following examples are now given.

## EXAMPLES

The following HPLC method was used in the analysis of the compounds, as specified in the synthetic examples set forth below. As used herein, the term "Rt" refers to the retention time (minutes) for the compound using the HPLC method specified below.

**LC-MS Method**

HPLC/MS was performed on a Waters X Terra RP 18 (4.6 x 50 mm, 3.5 μm) column using a Waters 2790 HPLC system equipped with a 996 Waters PDA detector and a Micromass mod. ZQ single quadrupole mass spectrometer, equipped with an electrospray (ESI) ion source. Mobile phase A was ammonium acetate 5 mM buffer (pH 5.5 with acetic acid / acetonitrile 95:5), and Mobile phase B was water / acetonitrile (5:95). Gradient from 10 to 90% B in 8 minutes, hold 90% B 2 min. UV detection at 220 nm and 254 nm. Flow rate 1 mL/min. Injection volume 10 μl. Full scan, mass range from 100 to 800 amu. Capillary voltage was 2.5 KV; Source temperature was 120°C; Cone was 10 V. Retention Times (LC-MS Rt) are given in minutes at 220 nm or 254 nm. Mass are given as m/z ratio.

**Example 1**

**3-Amino-furo[3,2-c]pyrazole-1,5-dicarboxylic acid 1-ethyl ester 5-propyl ester**

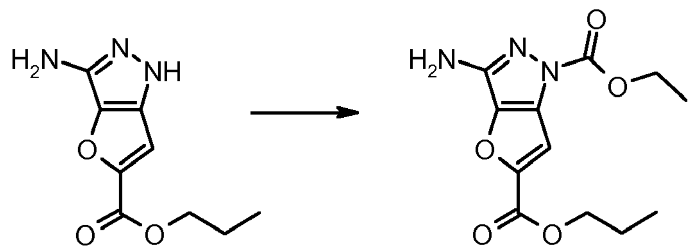

A solution of ethyl chlorocarbonate (4.90 mL, 51.7 mmol) in tetrahydrofuran (THF, 60 mL) was slowly added to a mixture of 3-Amino-1H-furo[3,2-c]pyrazole-5-carboxylic acid propyl ester (12.0 g, 50.2 mmol) and diisopropylethylamine (DIEA, 51.5 mL, 301 mmol) in THF (300 mL), maintaining the temperature between -5 and -10°C. The reaction was kept at the same temperature for 5 minutes then allowed to reach room temperature. The obtained mixture was evaporated to dryness under vacuum and the residue extracted with ethyl acetate (AcOEt) and water. The organic layer was separated, dried over sodium sulfate and evaporated to dryness. The resulting raw material was triturated with diethyl ether to give 13.7 g of the title compound as a colorless solid. $^1$H NMR (400 MHz, DMSO-D6) δ ppm 0.97 (t, 3 H) 1.35 (t, 3 H) 1.67 - 1.81 (m, 2 H) 4.28 (t, 2 H) 4.36 (q, 2 H) 6.35 (s, 2 H) 7.39 (s, 1 H); $[M+H]^+$ 282; HRMS ($ES^+$) calcd 282.1084, found 282.1075; LC-MS: Rt 4.92.

**Example 2**

**3-[(Thiophene-2-carbonyl)-amino]-furo[3,2-c]pyrazole-1,5-dicarboxylic acid 1-ethyl ester 5-propyl ester**

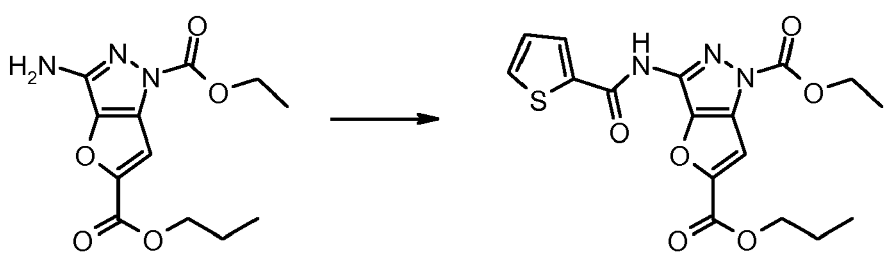

To a solution of 3-amino-furo[3,2-c]pyrazole-1,5-dicarboxylic acid 1-ethyl ester 5-propyl ester (500 mg, 1.606 mmol) and diisopropylethylamine (DIEA, 0.824 mL, 4.818 mmol) in tetrahydrofuran (THF, 20 mL) at 0 °C, 2-nitro-benzoyl chloride (0.318 mL, 2.409 mmol) was added. The reaction mixture was kept at the same temperature for 10 minutes then allowed to reach room temperature and let react overnight. The solvent was removed under vacuum, the residue was dissolved in dichloromethane (DCM) and washed with a 10 % solution of acetic acid (AcOH), water, saturated

$NaHCO_3$ and brine. The crude was purified on silica gel (eluant dichloromethane/methanol 96/4) yielding 512 mg (69 %) of the title compound. $^1H$ NMR (400 MHz, DMSO-D6) δ ppm 0.98 (t, 3 H) 1.41 (t, 3 H) 1.68 - 1.84 (m, 2 H) 4.31 (t, 2 H) 4.48 (q, 2 H) 7.28 (dd, 1 H) 7.53 (s, 1 H) 7.97 (dd, 1 H) 8.22 (dd, 1 H) 11.89 (s, 1 H); $[M+H]^+$ 392; HRMS ($ES^+$) calcd 392.0911, found 392.0915; LC-MS: Rt 7.48;

By operating in an analogous way, and by reacting 3-amino-furo[3,2-c]pyrazole-1,5-dicarboxylic acid 1-ethyl ester 5-propyl ester with the appropriate acyl chloride derivative, the following compounds in Example 3-46 were prepared:

## Example 3

**3-(4-Fluoro-benzoylamino)-furo[3,2-c]pyrazole-1,5-dicarboxylic acid 1-ethyl ester 5-propyl ester**

$^1H$ NMR (400 MHz, DMSO-D6) δ ppm 0.98 (t, 3 H) 1.42 (t, 3 H) 1.75 (tq, 2 H) 4.31 (t, 2 H) 4.49 (q, 2 H) 7.34 - 7.46 (m, 2 H) 7.53 (s, 1 H) 8.13 - 8.27 (m, 2 H) 11.83 (s, 1 H); $[M+H]^+$ 404; HRMS ($ES^+$) cald 404.1252, found 404.1266.

## Example 4

**3-(4-Morpholin-4-yl-benzoylamino)-furo[3,2-c]pyrazole-1,5-dicarboxylic acid 1-ethyl ester 5-propyl ester**

$[M+H]^+$ 471

## Example 5

**3-[(4-Bromo-thiophene-2-carbonyl)-amino]-furo[3,2-c]pyrazole-1,5-dicarboxylic acid 1-ethyl ester 5-propyl ester**

$^1H$ NMR (400 MHz, DMSO-D6) δ ppm 0.98 (t, 3 H) 1.41 (t, 3 H) 1.67 - 1.83 (m, 2 H) 4.31 (t, 2 H) 4.49 (q, 2 H) 7.53 (s, 1 H) 8.11 (d, 1 H) 8.23 (d, 1 H) 12.02 (s, 1 H); $[M+H]^+$ 470; HRMS ($ES^+$) cald 470.0016, found 469.9996

## Example 6

**3-[4-(4-Methyl-piperazin-1-yl)-benzoylamino]-furo[3,2-c]pyrazole-1,5-dicarboxylic acid 1-ethyl ester 5-propyl ester**

$^1H$ NMR (400 MHz, DMSO-D6) δ ppm 0.98 (t, 3 H) 1.41 (t, 3 H) 1.67 - 1.85 (m, 2 H) 2.27 (s, 3 H) 2.43 - 2.60 (m, 4 H) 3.23 - 3.44 (m, 4 H) 4.31 (t, 2 H) 4.48 (q, 2 H) 7.04 (d, 2 H) 7.51 (s, 1 H) 8.00 (d, 2 H) 11.35 - 11.61 (m, 1 H); $[M+H]^+$ 484; HRMS ($ES^+$) cald 484.2191, found 484.2167

## Example 7

**3-(3-Fluoro-benzoylamino)-furo[3,2-c]pyrazole-1,5-dicarboxylic acid 1-ethyl ester 5-propyl ester**

$[M+H]^+$ 404

## Example 8

**3-(2-Fluoro-benzoylamino)-furo[3,2-c]pyrazole-1,5-dicarboxylic acid 1-ethyl ester 5-propyl ester**

$[M+H]^+$ 404

## Example 9

**3-(4-Chloro-benzoylamino)-furo[3,2-c]pyrazole-1,5-dicarboxylic acid 1-ethyl ester 5**-propyl **ester**

$[M+H]^+$ 420

**Example 10**

**3-(4-Trifluoromethoxy-benzoylamino)-furo[3,2-c]pyrazole-1,5-dicarboxylic acid 1-ethyl ester 5-propyl ester**

$[M+H]^+$ 470

**Example 11**

**3-(4-Methoxy-benzoylamino)-furo[3,2-c]pyrazole-1,5-dicarboxylic acid 1-ethyl ester 5-propyl ester**

$[M+H]^+$ 416

**Example 12**

**3-(3-Methoxy-benzoylamino)-furo[3,2-c]pyrazole-1,5-dicarboxylic acid 1-ethyl ester 5-propyl ester**

$[M+H]^+$ 416

**Example 13**

**3-(2-Methoxy-benzoylamino)-furo[3,2-c]pyrazole-1,5-dicarboxylic acid 1-ethyl ester 5-propyl ester**

$[M+H]^+$ 416

**Example 14**

**3-(4-Cyano-benzoylamino)-furo[3,2-c]pyrazole-1,5-dicarboxylic acid 1-ethyl ester 5-propyl ester**

$[M+H]^+$ 411

**Example 15**

**3-[(Furan-2-carbonyl)-amino]-furo[3,2-c]pyrazole-1,5-dicarboxylic acid 1-ethyl ester 5-propyl ester**

$[M+H]^+$ 376

**Example 16**

**3-(4-Phenoxy-benzoylamino)-furo[3,2-c]pyrazole-1,5-dicarboxylic acid 1-ethyl ester 5-propyl ester**

$[M+H]^+$ 478

**Example 17**

**3-[4-(1-Methyl-piperidin-4-yloxy)-benzoylamino]-furo[3,2-c]pyrazole-1,5-dicarboxylic acid 1-ethyl ester 5-propyl ester**

$[M+H]^+$ 499

**Example 18**

**3-[4-(4-Methyl-piperazin-1-ylmethyl)-benzoylamino]-furo[3,2-c]pyrazole-1,5-dicarboxylic acid 1-ethyl ester 5-propyl ester**

$[M+H]^+$ 498

**Example 19**

**3-(4-Piperidin-1-ylmethyl-benzoylamino)-furo[3,2-c]pyrazole-1,5-dicarboxylic acid 1-ethyl ester 5-propyl ester**

$[M+H]^+$ 483

**Example 20**

**3-(4-Morpholin-4-ylmethyl-benzoylamino)-furo[3,2-c]pyrazole-1,5-dicarboxylic acid 1-ethyl ester 5-propyl ester**

$[M+H]^+$ 485

**Example 21**

**3-(4-Pyrrolidin-1-ylmethyl-benzoylamino)-furo[3,2-c]pyrazole-1,5-dicarboxylic acid 1-ethyl ester 5-propyl ester**

$[M+H]^+$ 469

**Example 22**

**3-(4-Azetidin-1-ylmethyl-benzoylamino)-furo[3,2-c]pyrazole-1,5-dicarboxylic acid 1-ethyl ester 5-propyl ester**

$[M+H]^+$ 455

**Example 23**

**3-[(1-Methyl-1H-pyrrole-2-carbonyl)-amino]-furo[3,2-c]pyrazole-1,5-dicarboxylic acid 1-ethyl ester 5-propyl ester**

$[M+H]^+$ 389

**Example 24**

**3-[(5-Methyl-isoxazole-3-carbonyl)-amino]-furo[3,2-c]pyrazole-1,5-dicarboxylic acid 1-ethyl ester 5-propyl ester**

$[M+H]^+$ 391

**Example 25**

**3-[(2,5-Dimethyl-2H-pyrazole-3-carbonyl)-amino]-furo[3,2-c]pyrazole-1,5-dicarboxylic acid 1-ethyl ester 5-propyl ester**

$[M+H]^+$ 404

**Example 26**

**3-[(2-Methyl-furan-3-carbonyl)-amino]-furo[3,2-c]pyrazole-1,5-dicarboxylic acid 1-ethyl ester 5-propyl ester**

$[M+H]^+$ 390

**Example 27**

**3-[(3-Methyl-thiophene-2-carbonyl)-amino]-furo[3,2-c]pyrazole-1,5-dicarboxylic acid 1-ethyl ester 5-propyl ester**

$[M+H]^+$ 406

## Example 28

**3-[(5-Methyl-thiophene-2-carbonyl)-amino]-furo[3,2-c]pyrazole-1,5-dicarboxylic acid 1-ethyl ester 5-propyl ester**

$[M+H]^+$ 406

## Example 29

**3-[(4,5-Dimethyl-furan-2-carbonyl)-amino]-furo[3,2-c]pyrazole-1,5-dicarboxylic acid 1-ethyl ester 5-propyl ester**

$[M+H]^+$ 404

## Example 30

**3-[(5-Chloro-thiophene-2-carbonyl)-amino]-furo[3,2-c]pyrazole-1,5-dicarboxylic acid 1-ethyl ester 5-propyl ester**

$[M+H]^+$ 426

## Example 31

**3-[(2,5-Dimethyl-furan-3-carbonyl)-amino]-furo[3,2-c]pyrazole-1,5-dicarboxylic acid 1-ethyl ester 5-propyl ester**

$[M+H]^+$ 404

## Example 32

**3-[(5-Methyl-furan-2-carbonyl)-amino]-furo[3,2-c]pyrazole-1,5-dicarboxylic acid 1-ethyl ester] 5-propyl ester**

$[M+H]^+$ 390

## Example 33

**3-[(4-Methyl-furan-2-carbonyl)-amino]-furo[3,2-c]pyrazole-1,5-dicarboxylic acid 1-ethyl ester] 5-propyl ester**

$[M+H]^+$ 390

## Example 34

**3-[(2-Methyl-2H-pyrazole-3-carbonyl)-amino]-furo[3,2-c]pyrazole-1,5-dicarboxylic acid 1-ethyl ester 5-propyl ester**

$[M+H]^+$ 390

## Example 35

**3-[(1-Methyl-1H-pyrazole-3-carbonyl)-amino]-furo[3,2-c]pyrazole-1,5-dicarboxylic acid 1-ethyl ester 5-propyl ester**

$[M+H]^+$ 390

**Example 36**

**3-[([1,2,3]Thiadiazole-4-carbonyl)-amino]-furo[3,2-c]pyrazole-1,5-dicarboxylic acid 1-ethyl ester 5-propyl ester**

$[M+H]^+$ 394

**Example 37**

**3-[(Furan-3-carbonyl)-amino]-furo[3,2-c]pyrazole-1,5-dicarboxylic acid 1-ethyl ester 5-propyl ester**

$[M+H]^+$ 376

**Example 38**

**3-[(Thiophene-3-carbonyl)-amino]-furo[3,2-c]pyrazole-1,5-dicarboxylic acid 1-ethyl ester 5-propyl ester**

$[M+H]^+$ 392

**Example 39**

**3-[(Thiazole-4-carbonyl)-amino]-furo[3,2-c]pyrazole-1,5-dicarboxylic acid 1-ethyl ester 5-propyl ester**

$[M+H]^+$ 393

**Example 40**

**3-[(1-Methyl-1H-imidazole-2-carbonyl)-amino]-furo[3,2-c]pyrazole-1,5-dicarboxylic acid 1-ethyl ester 5-propyl ester**

$[M+H]^+$ 390

**Example 41**

**3-[(1-Methyl-1H-imidazole-4-carbonyl)-amino]-furo[3,2-c]pyrazole-1,5-dicarboxylic acid 1-ethyl ester 5-propyl ester**

$[M+H]^+$ 390

**Example 42**

**3-[(1,3,5-Trimethyl-1H-pyrazole-4-carbonyl)-amino]-furo[3,2-c]pyrazole-1,5-dicarboxylic acid 1-ethyl ester 5-propyl ester**

$[M+H]^+$ 418

**Example 43**

**3-[(5-Morpholin-4-ylmethyl-furan-2-carbonyl)-amino]-furo[3,2-c]pyrazole-1,5-dicarboxylic acid 1-ethyl ester 5-propyl ester**

$[M+H]^+$ 475

**Example 44**

**3-Benzoylamino-furo[3,2-c]pyrazole-1,5-dicarboxylic acid 1-ethyl ester 5-propyl ester**

$[M+H]^+$ 386

**Example 45**

**3-[4-(2-Pyrrolidin-1-yl-ethoxy)-benzoylamino]-furo[3,2-c]pyrazole-1,5-dicarboxylic acid 1-ethyl ester 5-propyl ester**

$[M+H]^+$ 499

**Example 46**

**3-[(Pyridine-2-carbonyl)-amino]-furo[3,2-c]pyrazole-1,5-dicarboxylic acid 1-ethyl ester 5-propyl ester**

$[M+H]^+$ 387

**Example 47**

**3-[(Thiophene-2-carbonyl)-amino]-1H-furo[3,2-c]pyrazole-5-carboxylic acid**

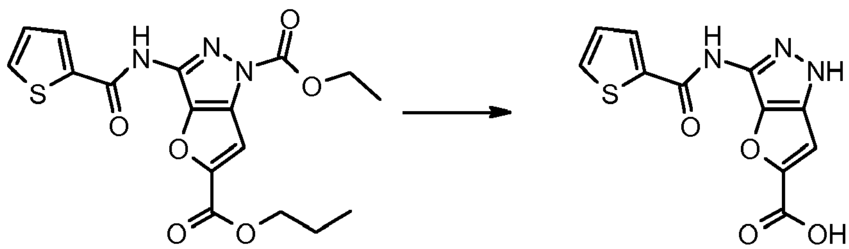

A suspension of 3-[(thiophene-2-carbonyl)-amino]-furo[3,2-c]pyrazole-1,5-dicarboxylic acid 1-ethyl ester 5-propyl ester (46.9 mmol, 19 g) in 40 mL of NaOH/H2O (1:1) and 100 mL of ethanol was heated at 70˚C, for 3 hours. The crude was then concentrated and the pH of the obtained suspension adjusted to pH=4 by using hydrochloric acid 37% and buffer pH 4. The aqueous layer was extracted with ethyl acetate (3x250 mL). The collected organic layers were washed with brine and dried over $Na_2SO_4$. The filtrate was evaporated to dryness to give 13.5 g of white solid, which was used into the next step without any further purification (yield 94%). $^1H$ NMR (400 MHz, DMSO-D6) δ ppm 7.25 (dd, 1 H) 7.39 (s, 1 H) 7.89 (dd, 1 H) 8.12 (dd, 1 H) 11.07 (s, 1 H) 12.61 (s, 1 H); $[M+H]^+$ 278; HRMS ($ES^+$) cald 278.023, found 278.038.

Analogously, the following compounds in Examples 48-90 were prepared by using the 3-(carbonyl-amino)-furo [3,2-c]pyrazole-1,5-dicarboxylic acid 1-ethyl ester 5-propyl ester derivatives from Example 47:

**Example 48**

**3-(4-Fluoro-benzoylamino)-1H-furo[3,2-c]pyrazole-5-carboxylic acid**

$^1H$ NMR (400 MHz, DMSO-D6) δ ppm 6.85 (s, 1 H) 7.27 - 7.60 (m, 2 H) 7.84 - 8.48 (m, 2 H) 10.89 (s, 1 H) 12.23 (s, 1 H); $[M+H]^+$ 290; HRMS ($ES^+$) cald 290.0572, found 290.0568

**Example 49**

**3-(4-Morpholin-4-yl-benzoylamino)-1H-furo[3,2-c]pyrazole-5-carboxylic acid**

$^1H$ NMR (400 MHz, DMSO-D6) δ ppm 3.05 - 3.55 (m, 4 H) 3.67 - 3.88 (m, 4 H) 6.82 (s, 1 H) 7.04 (d, 2 H) 7.98 (d, 2 H) 10.54 (s, 1 H) 12.14 (s, 1 H); $[M+H]^+$ 357; HRMS ($ES^+$) cald 357.1193, found 357.1188

**Example 50**

**3-[(4-Bromo-thiophene-2-carbonyl)-amino]-1H-furo[3,2-c]pyrazole-5-carboxylic acid**

$^1H$ NMR (400 MHz, DMSO-D6) δ ppm 7.13 (s, 1 H) 8.03 (d, 1 H) 8.14 (d, 1 H) 11.12 (s, 1 H) 12.47 (s, 1 H);

$[M+H]^+$ 356; HRMS ($ES^+$) cald 355.9335, found 355.9348; LC-MS: RT 2.31

**Example 51**

**3-[4-(4-Methyl-piperazin-1-yl)-benzoylamino]-1H-furo[3,2-c]pyrazole-5-carboxylic acid**

$^1$H NMR (400 MHz, DMSO-D6) δ ppm 2.25 (s, 3 H) 2.50 - 2.56 (m, 4 H) 3.14 - 3.60 (m, 4 H) 6.97 - 7.06 (m, 3 H) 7.95 (d, 2 H) 10.53 (s, 1 H) 12.25 (s, 1 H); $[M+H]^+$ 370; HRMS ($ES^+$) cald 370.151o, found 370.1513.

**Example 52**

**3-(3-Fluoro-benzoylamino)-1H-furo[3,2-c]pyrazole-5-carboxylic acid**

$[M+H]^+$ 290

**Example 53**

**3-(2-Fluoro-benzoylamino)-1H-furo[3,2-c]pyrazole-5-carboxylic acid**

$[M+H]^+$ 290

**Example 54**

**3-(4-Chloro-benzoylamino)-1H-furo[3,2-c]pyrazole-5-carboxylic acid**

$^1$H NMR (400 MHz, DMSO-D6) δ ppm 7.41 (s, 1 H) 7.64 (d, 2 H) 8.08 (d, 2 H) 11.09 (s, 1 H) 12.63 (s, 1 H) 13.38 (br. s, 1 H); $[M+H]^+$ 306; HRMS ($ES^+$) cald 306.0276, found 306.0272

**Example 55**

**3-(4-Trifluoromethoxy-benzoylamino)-1H-furo[3,2-c]pyrazole-5-carboxylic acid**

$[M+H]^+$ 356

**Example 56**

**3-(4-Methoxy-benzoylamino)-1H-furo[3,2-c]pyrazole-5-carboxylic acid**

$^1$H NMR (400 MHz, DMSO-D6) δ ppm 3.84 - 3.92 (m, 3 H) 7.09 (d, 2 H) 7.39 (s, 1 H) 8.06 (d, 2 H) 10.81 (s, 1 H) 12.55 (s, 1 H) 12.77 - 14.07 (br. s, 1 H); $[M+H]^+$ 302; HRMS ($ES^+$) cald 302.0771, found 302.0771

**Example 57**

**3-(3-Methoxy-benzoylamino)-1H-furo[3,2-c]pyrazole-5-carboxylic acid**

$^1$H NMR (400 MHz, DMSO-D6) δ ppm 3.86 (s, 3 H) 7.12 - 7.24 (m, 1 H) 7.40 (s, 1 H) 7.42 - 7.49 (m, 1 H) 7.61 - 7.73 (m, 2 H) 10.99 (s, 1 H) 12.61 (s, 1 H) 12.85 - 14.09 (br. s, 1 H); $[M+H]^+$302; HRMS ($ES^+$) cald 302.0771, found 302.0770

**Example 58**

**3-(2-Methoxy-benzoylamino)-1H-furo[3,2-c]pyrazole-5-carboxylic acid**

$[M+H]^+$ 302

**Example 59**

**3-(4-Cyano-benzoylamino)-1H-furo[3,2-c]pyrazole-5-carboxylic acid**

$[M+H]^+$ 297

**Example 60**

**3-[(Furan-2-carbonyl)-amino]-1H-furo[3,2-c]pyrazole-5-carboxylic acid**

$^1$H NMR (400 MHz, DMSO-D6) δ ppm 6.69 (dd, J=3.48, 1.77 Hz, 1 H) 7.19 (s, 1 H) 7.43 (d, J=3.41 Hz, 1 H) 7.43 (dd, J=3.41, 1.77 Hz, 1 H) 7.93 (dd, J=1.65, 0.67 Hz, 1 H) 7.93 (dd, J=1.65, 0.67 Hz, 1 H) 10.79 (s, 1 H) 12.46 (s, 1 H); $[M+H]^+$ 262; HRMS ($ES^+$) cald 262.0458, found 262.0445

**Example 61**

**3-(4-Phenoxy-benzoylamino)-1H-furo[3,2-c]pyrazole-5-carboxylic acid**

[M+H]+ 364

**Example 62**

**3-[4-(1-Methyl-piperidin-4-yloxy)-benzoylamino]-1H-furo[3,2-c]pyrazole-5-carboxylic acid**

$[M+H]^+$ 385

**Example 63**

**3-[4-(4-Methyl-piperazin-1-ylmethyl)-benzoylamino]-1H-furo[3,2-c]pyrazole-5-carboxylic acid**

$[M+H]^+$ 384

**Example 64**

**3-(4-Piperidin-1-ylmethyl-benzoylamino)-1H-furo[3,2-c]pyrazole-5-carboxylic acid**

$[M+H]^+$ 369

**Example 65**

**3-(4-Morpholin-4-ylmethyl-benzoylamino)-1H-furo[3,2-c]pyrazole-5-carboxylic acid salt with formic acid**

$^1$H NMR (400 MHz, DMSO-D6) δ ppm 2.47 - 2.60 (m, 4 H) 3.15 - 3.51 (s, 2 H) 3.56 - 3.80 (m, 4 H) 7.40 (s, 1 H) 7.51 (d, 2 H) 8.04 (d, 2 H) 10.95 (s, 1 H) 12.59 (s, 1 H) $[M+H]^+$ 371; HRMS ($ES^+$) cald 371.1350, found 371.1357.

**Example 66**

**3-(4-Pyrrolidin-1-ylmethyl-benzoylamino)-1H-furo[3,2-c]pyrazole-5-carboxylic acid**

$[M+H]^+$ 355

**Example 67**

**3-(4-Azetidin-1-ylmethyl-benzoylamino)-1H-furo[3,2-c]pyrazole-5-carboxylic acid**

$[M+H]^+$ 341

**Example 68**

**3-[(1-Methyl-1H-pyrrole-2-carbonyl)-amino]-1H-furo[3,2-c]pyrazole-5-carboxylic acid**

$^{1}H$ NMR (400 MHz, DMSO-D6) δ ppm 3.92 (s, 3 H) 6.11 (dd, 1 H) 7.04 (dd, 1 H) 7.18 (dd, 1 H) 7.38 (s, 1 H) 10.43 (s, 1 H) 12.49 (s, 1 H); $[M+H]^+$ 275; HRMS ($ES^+$) cald 275.0775, found 275.0784

**Example 69**

**3-[(5-Methyl-isoxazole-3-carbonyl)-amino]-1H-furo[3,2-c]pyrazole-5-carboxylic acid**

$[M+H]^+$ 277

**Example 70**

**3-[(2,5-Dimethyl-2H-pyrazole-3-carbonyl)-amino]-1H-furo[3,2-c]pyrazole-5-carboxylic a**cid

$[M+H]^+$ 290

**Example 71**

**3-[(2-Methyl-furan-3-carbonyl)-amino]-1H-furo[3,2-c]pyrazole-5-carboxylic acid**

$[M+H]^+$ 276

**Example 72**

**3-[(3-Methyl-thiophene-2-carbonyl)-amino]-1H-furo[3,2-c]pyrazole-5-carboxylic acid**

$[M+H]^+$ 292

**Example 73**

**3-[(5-Methyl-thiophene-2-carbonyl)-amino]-1H-furo[3,2-c]pyrazole-5-carboxylic acid**

$[M+H]^+$ 292

**Example 74**

**3-[(4,5-Dimethyl-furan-2-carbonyl)-amino]-1H-furo[3,2-c]pyrazole-5-carboxylic acid**

$[M+H]^+$ 290

**Example 75**

**3-[(5-Chloro-thiophene-2-carbonyl)-amino]-1H-furo[3,2-c]pyrazole-5-carboxylic acid**

$[M+H]^+$ 312

**Example 76**

**3-[(2,5-Dimethyl-furan-3-carbonyl)-amino]-1H-furo[3,2-c]pyrazole-5-carboxylic acid**

$[M+H]^+$ 290

**Example 77**

**3-[(5-Methyl-furan-2-carbonyl)-amino]-1H-furo[3,2-c]pyrazole-5-carboxylic acid**

$[M+H]^+$ 276

**Example 78**

**3-[(4-Methyl-furan-2-carbonyl)-amino]-1H-furo[3,2-c]pyrazole-5-carboxylic acid**

$[M+H]^+$ 276

**Example 79**

**3-[(2-Methyl-2H-pyrazole-3-carbonyl)-amino]-1H-furo[3,2-c]pyrazole-5-carboxylic acid**

$[M+H]^+$ 276

**Example 80**

**3-[(1-Methyl-1H-pyrazole-3-carbonyl)-amino]-1H-furo[3,2-c]pyrazole-5-carboxylic acid**

$[M+H]^+$ 276

**Example 81**

**3-[([1,2,3]Thiadiazole-4-carbonyl)-amino]-1H-furo[3,2-c]pyrazole-5-carboxylic acid**

$[M+H]^+$ 280

**Example 82**

**3-[(Furan-3-carbonyl)-amino]-1H-furo[3,2-c]pyrazole-5-carboxylic acid**

$[M+H]^+$ 262

**Example 83**

**3-[(Thiophene-3-carbonyl)-amino]-1H-furo[3,2-c]pyrazole-5-carboxylic acid**

$[M+H]^+$ 278

**Example 84**

**3-[(Thiazole-4-carbonyl)-amino]-1H-furo[3,2-c]pyrazole-5-carboxylic acid**

$[M+H]^+$ 279

**Example 85**

**3-[(1-Methyl-1H-imidazole-4-carbonyl)-amino]-1H-furo[3,2-c]pyrazole-5-carboxylic acid**

$[M+H]^+$ 276

**Example 86**

**3-[(1,3,5-Trimethyl-1 H-pyrazole-4-carbonyl)-amino]-1H-furo[3,2-c]pyrazole-5-carboxylic acid**

$[M+H]^+$ 304

**Example 87**

**3-[(5-Morpholin-4-ylmethyl-furan-2-carbonyl)-amino]-1H-furo[3,2-c]pyrazole-5-carboxylic acid**

$[M+H]^+$ 361

**Example 88**

**3-Benzoylamino-1H-furo[3,2-c]pyrazole-5-carboxylic acid**

$^1H$ NMR (400 MHz, DMSO-D6) δ ppm 7.40 (s, 1 H) 7.52 - 7.59 (m, 2 H) 7.59 - 7.68 (m, 1 H) 8.04 - 8.13 (m, 2 H) 10.98 (s, 1 H) 12.60 (s, 1 H) 13.36 (br. s, 1 H); $[M+H]^+$ 272; HRMS ($ES^+$) cald 272.0666, found 272.0674. **3-[4-(2-Pyrrolidin-1-yl-ethoxy)-benzoylamino]-1H-furo[3,2-c]pyrazole-5-carboxylic acid** $[M+H]^+$ 385

**Example 89**

**3-(3-Chloro-benzoylamino)-1H-furo[3,2-c]pyrazole-5-carboxylic acid**

$^1H$ NMR (400 MHz, DMSO-D6) δ ppm 7.39 (s, 1 H) 7.56 - 7.64 (m, 1 H) 7.64 - 7.76 (m, 1 H) 8.00 - 8.08 (m, 1 H) 8.09 - 8.18 (m, 1 H) 11.13 (s, 1 H) 12.62 (s, 1 H) 12.87 - 13.99 (m, 1 H); $[M+H]^+$ 306; HRMS ($ES^+$) cald 306.0276, found 306.0272.

**Example 90**

**3-[(Pyridine-2-carbonyl)-amino]-1H-furo[3,2-c]pyrazole-5-carboxylic acid**

$[M+H]^+$ 273.

**Example 91**

**3-[(Thiophene-2-carbonyl)-amino]-1H-furo[3,2-c]pyrazole-5-carboxylic acid (1-methyl-1-phenyl-ethyl)-amide (1)**

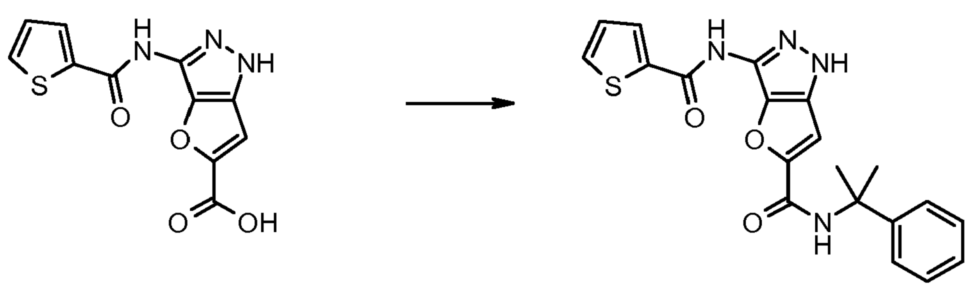

To a suspension of 3-[(thiophene-2-carbonyl)-amino]-1H-furo[3,2-c]pyrazole-5-carboxylic acid (0.47 mMol, 0.130 g) in dry dichloromethane (5 mL) at - 5 ˚C, N,N-diisopropylethylamine (DIEA, 0.39 mL, 3.74 mMol) and ethyl chloroformate (0.15 mL, 1.40 mMol) were added. The reaction mixture was stirred until reaching room temperature (about one hour), then cumylamine (200 mg, 1.4 mMol) was added. The mixture was stirred at room temperature for 24 hours, and then diluted with ethyl acetate and washed twice with a saturated solution of sodium bicarbonate and twice with brine. The organic layer was collected, and evaporated to dryness, and re-dissolved in dichloromethane (10

mL). To this solution hexane (50 mL) was added, and white precipitate so obtained was collected and washed with hexane. This was dissolved in methanol-triethylamine (8 : 2, 10 mL) and stirred at room temperature for 4 hours. The solvent was evaporated and the crude purified by flash column chromatography on silica gel, eluting with ethyl acetate - hexane 3 :1). 70 mg of the title compound was so obtained as a colorless solid. $^{1}$H NMR (400 MHz, DMSO-D6) δ ppm 1.70 (s, 6 H) 7.19 (ddd, 1 H) 7.24 (dd, 1 H) 7.27 - 7.35 (m, 3 H) 7.36 - 7.45 (m, 2 H) 7.89 (dd, 1 H) 8.10 (dd, 1 H) 8.30 (s, 1 H) 10.99 (s, 1 H) 12.54 (s, 1 H); $[M+H]^+$ 395; HRMS ($ES^+$) cald 395.1172, found 395.1177.

Analogously, the following compounds in Examples 92-163 were prepared by using the appropriate 3-(carbonyl-amino)-furo[3,2-c]pyrazole-5-carboxylic acid and the appropriate amine:

**Example 92**

**2) 3-(4-Morpholin-4-ylmethyl-benzoylamino)-1H-furo[3,2-c]pyrazole-5-carboxylic acid[1-(2-fluoro-phenyl)-1-methyl-ethyl]-amide**

$^{1}$H NMR (400 MHz, DMSO-D6) δ ppm 1.76 (s, 6 H) 2.36 - 2.45 (m, 4 H) 3.57 (s, 2 H) 3.58 - 3.66 (m, 4 H) 7.04 - 7.21 (m, 2 H) 7.23 - 7.31 (m, 1 H) 7.35 (s, 1 H) 7.36 - 7.43 (m, 1 H) 7.49 (d, 2 H) 8.02 (d, 2 H) 8.31 (s, 1 H) 10.79 - 11.04 (m, 2 H); $[M+H]^+$ 506; HRMS ($ES^+$) calcd 506.2198, found 506.2216; LC-MS: RT 3.81

**Example 93**

**3-[4-(4-Methyl-piperazin-1-yl)-benzoylamino]-1H-furo[3,2-c]pyrazole-5-carboxylic acid (1-phenyl-cyclopropyl)-amide**

$^{1}$H NMR (500 MHz, DMSO-D6) δ ppm 1.21 - 1.40 (m, 4 H) 2.26 (s, 3 H) 2.44 - 2.51 (m, 4 H) 3.27 - 3.44 (m, 4 H) 7.04 (d, 2 H) 7.19 (tt, 1 H) 7.22 - 7.26 (m, 2 H) 7.28 - 7.33 (m, 2 H) 7.35 (s, 1 H) 7.96 (d, 2 H) 9.20 (s, 1 H) 10.55 (s, 1 H) 12.46 (s, 1 H); $[M+H]^+$ 485; HRMS ($ES^+$) calcd 485.2296, found 485.2300; LC-MS: RT 3.21.

**Example 94**

**3-[(4-fluorobenzoyl)amino]-N-(1-methyl-1-phenylethyl)-1H-furo[3,2-c]pyrazole-5-carboxamide**

$^{1}$H NMR (400 MHz, DMSO-D6) δ ppm 1.69 (s, 6 H) 7.16 - 7.45 (m, 8 H) 8.07 - 8.21 (m, 2 H) 8.29 (s, 1 H) 10.95 (s, 1 H) 12.53 (s, 1 H); $[M+H]^+$ 407; HRMS ($ES^+$) cald 407.1514, found 407.1520

**Example 95**

**3-[(4-fluorobenzoyl)amino]-N-(1-phenylcyclopropyl)-1H-furo[3,2-c]pyrazole-5-carboxamide**

$^{1}$H NMR (400 MHz, DMSO-D6) δ ppm 1.22 - 1.36 (m, 4H) 7.11 - 7.33 (m, 5 H) 7.33 - 7.45 (m, 3 H) 8.04 - 8.22 (m, 2 H) 9.20 (s, 1 H) 10.93 (s, 1 H) 12.54 (s, 1 H); $[M+H]^+$ 405; HRMS ($ES^+$) calcd 405.1357, found 405.1357; LC-MS: RT 5.06

**Example 96**

**N-(1-ethyl-1-phenylpropyl)-3-[(4-fluorobenzoyl)amino]-1H-furo[3,2-c]pyrazole-5-carboxamide**

$^{1}$H NMR (400 MHz, DMSO-D6) δ ppm 0.71 (t, 6H) 1.95 - 2.08 (m, 2H) 2.15 - 2.30 (m, 2H) 7.15 - 7.26 (m, 1 H) 7.25 - 7.45 (m, 7 H) 7.82 (s, 1 H) 8.05 - 8.25 (m, 2 H) 10.96 (s, 1 H) 12.54 (s, 1 H); $[M+H]^+$ 435; HRMS ($ES^+$) calcd 435.1827, found 435.1844. LC-MS: RT 5.90

**Example 97**

**3-[(4-morpholin-4-ylbenzoyl)amino]-N-(1-phenylcyclopropyl)-1H-furo[3,2-c]pyrazole-5-carboxamide**

$^{1}$H NMR (400 MHz, DMSO-D6) δ ppm 1.19 - 1.36 (m, 4 H) 3.23 - 3.31 (m, 4 H) 3.73 - 3.80 (m, 4 H) 7.04 (d, 2 H) 7.17 (tt, 1 H) 7.23 (dd, 2 H) 7.27 (dd, 2 H) 7.33 (s, 1 H) 7.97 (d, 2 H) 9.20 (s, 1 H) 10.57 (s, 1 H) 12.46 (s, 1 H); $[M+H]^+$ 472; HRMS ($ES^+$) calcd 472.1979, found 472.1973

**Example 98**

**N-(1-ethyl-1-phenylpropyl)-3-[(4-morpholin-4-ylbenzoyl)amino]-1H-furo[3,2-c]pyrazole-5-carboxamide**

$^1$H NMR (400 MHz, DMSO-D6) δ ppm 0.71 (t, 6 H) 1.94 - 2.07 (m, 2 H) 2.17 - 2.29 (m, 2 H) 3.24 - 3.31 (m, 4 H) 3.72 - 3.80 (m, 4 H) 7.04 (d, 2 H) 7.20 (tt, 1 H) 7.28 - 7.40 (m, 5 H) 7.83 (s, 1 H) 7.97 (d, 2 H) 10.60 (s, 1 H) 12.45 (s, 1 H); $[M+H]^+$ 502; HRMS ($ES^+$) calcd 502.2449, found 502.2437; LC-MS: RT 5.54

**Example 99**

**3-[(4-fluorobenzoyl)amino]-N-(1-phenylcyclopentyl)-1H-furo[3,2-c]pyrazole-5-carboxamide**

$^1$H NMR (400 MHz, DMSO-D6) δ ppm 1.66 - 1.86 (m, 4 H) 1.93 - 2.15 (m, 2 H) 2.50 - 2.54 (m, 2 H) 7.15 - 7.22 (m, 1 H) 7.26 - 7.34 (m, 3 H) 7.34 - 7.46 (m, J=15.00 Hz, 4 H) 8.04 - 8.22 (m, 2 H) 8.39 (s, 1 H) 10.93 (s, 1 H) 12.53 (s, 1 H); $[M+H]^+$ 433; HRMS ($ES^+$) calcd 433.1670, found 433.1677. LC-MS: RT 5.79

**Example 100**

**N-(1-methyl-1-phenylethyl)-3-{[4-(4-methylpi perazin-1-yl)benzoyl]amino}-1H-furo[3,2-c]pyrazole-5-carboxamide**

$^1$H NMR (400 MHz, DMSO-D6) δ ppm 1.70 (s, 6 H) 2.27 (s, 3 H) 2.49 (s, 4 H) 3.31 (s, 4 H) 7.02 (d, J=9.15 Hz, 2 H) 7.16 - 7.22 (m, 1 H) 7.27 - 7.34 (m, 2 H) 7.30 (s, 1 H) 7.37 - 7.44 (m, 2 H) 7.96 (d, J=9.02 Hz, 2 H) 8.30 (s, 1 H) 10.56 (s, 1 H) 12.43 (s, 1 H); $[M+H]^+$ 487; HRMS ($ES^+$) calcd 487.2452, found 487.2453. LC-MS: RT 3.36

**Example 101**

**N-(1-ethyl-1-phenylpropyl)-3-{[4-(4-methylpiperazin-1-yl)benzoyl]amino}-1H-furo[3,2-c]pyrazole-5-carboxamide**

$^1$H NMR (400 MHz, DMSO-D6) δ ppm 0.71 (t, J=7.38 Hz, 6 H) 1.93 - 2.05 (m, 2 H) 2.16 - 2.27 (m, 2 H) 2.31 - 2.38 (m, 3 H) 2.43 - 2.59 (m, 4 H) 3.13 - 3.46 (m, 4 H) 7.03 (d, J=9.27 Hz, 2 H) 7.17 - 7.24 (m, 1 H) 7.32 (t, J=7.74 Hz, 2 H) 7.34 - 7.39 (m, 2 H) 7.35 (s, 1 H) 7.83 (s, 1 H) 7.96 (d, J=9.02 Hz, 2 H) 10.59 (s, 1 H) 12.45 (s, 1 H); $[M+H]^+$ 515; LC-MS: RT 3.98

**Example 102**

**3-[(3-fluorobenzoyl)amino]-N-(1-methyl-1-phenylethyl)-1H-furo[3,2-c]pyrazole-5-carboxamide**

$^1$H NMR (500 MHz, DMSO-D6) δ ppm 1.71 (s, 6 H) 7.21 (tt, 1 H) 7.32 (dd, 2 H) 7.36 (s, 1 H) 7.38 - 7.44 (m, 2 H) 7.46 - 7.55 (m, 1 H) 7.57 - 7.72 (m, 1 H) 7.79 - 8.03 (m, J=19.49 Hz, 2 H) 8.28 (s, 1 H) 11.04 (s, 1 H) 12.57 (s, 1 H); $[M+H]^+$ 407; HRMS ($ES^+$) calcd 407.1514, found 407.1520; LC-MS: RT 5.41

**Example 103**

**3-[(4-chlorobenzoyl)amino]-N-(1-methyl-1-phenylethyl)-1H-furo[3,2-c]pyrazole-5-carboxamide**

$^1$H NMR (400 MHz, DMSO-D6) δ ppm 1.70 (s, 6 H) 7.19 (tt, 1 H) 7.28 - 7.34 (m, 3 H) 7.38 - 7.43 (m, 2 H) 7.64 (d, 2 H) 8.07 (d, 2 H) 8.30 (s, 1 H) 11.02 (s, 1 H) 12.54 (s, 1 H); $[M+H]^+$ 423

**Example 104**

**3-[(2-fluorobenzoyl)amino]-N-(1-methyl-1-phenylethyl)-1H-furo[3,2-c]pyrazole-5-carboxamide**

$^1$H NMR (400 MHz, DMSO-D6) δ ppm 1.70 (s, 6 H) 7.15 - 7.24 (m, 1 H) 7.27 - 7.45 (m, 7 H) 7.56 - 7.67 (m, 1 H) 7.70 -7.80 (m, 1 H) 8.28 (s, 1 H) 10.87 (s, 1 H) 12.52 (s, 1 H); $[M+H]^+$ 407; HRMS ($ES^+$) calcd 407.1514, found 407.1534; LC-MS: RT 5.14.

**Example 105**

**N-(1-methyl-1-phenylethyl)-3-{[4-(trifluoromethoxy)benzoyl]amino}-1H-furo[3,2-c]pyrazole-5-carboxamide**

$^1$H NMR (400 MHz, DMSO-D6) δ ppm 1.54 (none, 1 H) 1.70 (s, 6 H) 7.20 (tt, 1 H) 7.27 - 7.36 (m, 3 H) 7.40 (dd, 2 H) 7.56 (d, 2 H) 8.18 (d, 2 H) 8.29 (s, 1 H) 11.07 (s, 1 H) 12.55 (br. s., 1 H); $[M+H]^+$ 473; LC-MS: RT 6.14

**Example 106**

**3-[(2-methoxybenzoyl)amino]-N-(1-methyl-1-phenylethyl)-1H-furo[3,2-c]pyrazole-5-carboxamide**

$^1$H NMR (400 MHz, DMSO-D6) δ ppm 1.70 (s, 6 H) 3.97 (s, 3 H) 7.08 - 7.15 (m, 1 H) 7.17 - 7.26 (m, 2 H) 7.27 - 7.36 (m, 3 H) 7.37 - 7.44 (m, 2 H) 7.53 - 7.61 (m, 1 H) 7.84 (dd, 1 H) 8.29 (s, 1 H) 10.33 (s, 1 H) 12.48 (br. s., 1 H); $[M+H]^+$ 419; LC-MS: RT 5.45

**Example 107**

**3-[(3-methoxybenzoyl)amino]-N-(1-methyl-1-phenylethyl)-1H-furo[3,2-c]pyrazole-5-carboxamide**

$^1$H NMR (400 MHz, DMSO-D6) δ ppm 1.70 (s, 6 H) 3.86 (s, 3 H) 7.15 - 7.23 (m, 2 H) 7.27 - 7.34 (m, 3 H) 7.38 - 7.43 (m, 2 H) 7.46 (dd, 1 H) 7.58 - 7.70 (m, 2 H) 8.31 (s, 1 H) 10.91 (s, 1 H) 12.53 (br. s., 1 H); $[M+H]^+$ 419; HRMS ($ES^+$) calcd 419.1714, found 419.1712; LC-MS: RT 5.36

**Example 108**

**3-[(4-methoxybenzoyl)amino]-N-(1-methyl-1-phenylethyl)-1H-furo[3,2-c]pyrazole-5-carboxamide**

$^1$H NMR (400 MHz, DMSO-D6) δ ppm 1.70 (s, 6 H) 3.86 (s, 3 H) 7.09 (d, 2 H) 7.19 (tt, 1 H) 7.27 - 7.35 (m, 3 H) 7.40 (dd, 2 H) 8.05 (d, 2 H) 8.31 (s, 1 H) 10.74 (s, 1 H) 12.48 (br. s., 1 H); $[M+H]^+$ 419; HRMS ($ES^+$) calcd 419.1714, found 419.1704; LC-MS: RT 5.26

**Example 109**

**N-(1-phenylcyclopropyl)-3-[(2-thienylcarbonyl)amino]-1H-furo[3,2-c]pyrazole-5-carboxamide**

$^1$H NMR (400 MHz, DMSO-D6) δ ppm 1.22 - 1.33 (m, 4 H) 7.17 (tt, 1 H) 7.20 - 7.32 (m, 5 H) 7.34 (s, 1 H) 7.89 (dd, 1 H) 8.11 (dd, 1 H) 9.21 (s, 1 H) 10.97 (s, 1 H) 12.56 (br. s., 1 H); $[M+H]^+$ 393; HRMS ($ES^+$) calcd 393.1016, found 393.1019

**Example 110**

**N-[1-(4-chlorophenyl)-1-methylethyl]-3-[(2-thienylcarbonyl)amino]-1H-furo[3,2-c]pyrazole-5-carboxamide**

$^1$H NMR (400 MHz, DMSO-D6) δ ppm 1.68 (s, 6 H) 7.25 (dd, 1 H) 7.31 (s, 1 H) 7.36 (d, 2 H) 7.40 (d, 2 H) 7.89 (dd, 1 H) 8.10 (dd, 1 H) 8.38 (s, 1 H) 10.98 (s, 1 H) 12.54 (br. s., 1 H); $[M+H]^+$ 429; HRMS ($ES^+$) calcd 429.0783, found 429.0790; LC-MS: RT 5.75

**Example 111**

**N-[1-(4-chlorophenyl)-1-methylethyl]-3-[(4-fluorobenzoyl)amino]-1H-furo[3,2-c]pyrazole-5-carboxamide**

$^1$H NMR (400 MHz, DMSO-D6) δ ppm 1.68 (s, 6 H) 7.29 - 7.45 (m, 7 H) 8.08 - 8.21 (m, 2 H) 8.39 (s, 1 H) 10.95 (s, 1 H) 12.54 (br. s., 1 H); $[M+H]^+$ 441; HRMS ($ES^+$) calcd 441.1124, found 441.1143.

**Example 112**

**3-[(4-cyanobenzoyl)amino]-N-(1-methyl-1-phenylethyl)-1H-furo[3,2-c]pyrazole-5-carboxamide**

$[M+H]^+$ 414; LC-MS: RT 5.66

**Example 113**

**N-[1-(2-chlorophenyl)-1-methylethyl]-3-[(2-thienylcarbonyl)amino]-1H-furo[3,2-c]pyrazole-5-carboxamide**

$^1H$ NMR (400 MHz, DMSO-D6) δ ppm 1.80 (s, 6 H) 7.21 - 7.27 (m, 2 H) 7.30 - 7.36 (m, 3 H) 7.56 (dd, 1 H) 7.88 (dd, 1 H) 8.09 (dd, 1 H) 8.33 (s, 1 H) 10.95 (s, 1 H) 12.52 (s, 1 H); $[M+H]^+$ 429; HRMS ($ES^+$) calcd 429.0783, found 429.0780; LC-MS: RT 5.55

**Example 114**

**N-(1-phenylcyclopentyl)-3-[(2-thienylcarbonyl)amino]-1H-furo[3,2-c]pyrazole-5-carboxamide**

$^1H$ NMR (400 MHz, DMSO-D6) δ ppm 1.66 - 1.89 (m, 6 H) 1.96 - 2.11 (m, 2 H) 7.19 (tt, 1 H) 7.24 (dd, 1 H) 7.27 - 7.33 (m, 3 H) 7.40 - 7.45 (m, 2 H) 7.90 (dd, 1 H) 8.09 (dd, 1 H) 8.39 (s, 1 H) 10.95 (s, 1 H) 12.54 (s, 1 H); $[M+H]^+$ 421; LC-MS: RT 5.85

**Example 115**

**N-(1-ethyl-1-phenylpropyl)-3-[(2-thienylcarbonyl)amino]-1H-furo[3,2-c]pyrazole-5-carboxamide**

$^1H$ NMR (400 MHz, DMSO-D6) δ ppm 0.71 (t, 6 H) 2.02 (dq, 2 H) 2.22 (dq, 2 H) 7.17 - 7.26 (m, 2 H) 7.28 - 7.39 (m, 5 H) 7.82 (s, 1 H) 7.90 (dd, 1 H) 8.11 (dd, 1 H) 11.00 (s, 1 H) 12.55 (s, 1 H); $[M+H]^+$ 423; HRMS ($ES^+$) calcd 423.1485, found 423.1490; LC-MS: RT 5.99

**Example 116**

**N-[1-(3-chlorophenyl)-1-methylethyl]-3-{[4-(4-methylpiperazin-1-yl)benzoyl]amino}-1H-furo[3,2-c]pyrazole-5-carboxamide**

$^1H$ NMR (400 MHz, DMSO-D6) δ ppm 1.68 (s, 6 H) 2.27 - 2.37 (m, 3 H) 2.54 - 2.63 (m, 4 H) 3.23 - 3.40 (m, 4 H) 7.04 (d, 2 H) 7.23 - 7.43 (m, 5 H) 7.95 (d, 2 H) 8.43 (s, 1 H) 10.58 (s, 1 H) 12.45 (s, 1 H); $[M+H]^+$ 522; HRMS ($ES^+$) calcd 521.2062, found 521.205

**Example 117**

**3-{[4-(4-methylpiperazin-1-yl)benzoyl]amino}-N-(1-phenylcyclopentyl)-1H-furo[3,2-c]pyrazole-5-carboxamide**

$^1H$ NMR (400 MHz, DMSO-D6) δ ppm 1.68 - 1.88 (m, 4 H) 1.94 - 2.14 (m, 2 H) 2.28 (s, 3 H) 2.48 - 2.57 (m, 6 H) 3.26 - 3.38 (m, 4 H) 7.03 (d, 2 H) 7.19 (tt, 1 H) 7.26 - 7.33 (m, 3 H) 7.42 (dd, 2 H) 7.94 (d, 2 H) 8.39 (s, 1 H) 10.53 (s, 1 H) 12.43 (s, 1 H); $[M+H]^+$ 513

**Example 118**

**3-(2-furoylamino)-N-(1-methyl-1-phenylethyl)-1H-furo[3,2-c]pyrazole-5-carboxamide** $^1H$ NMR (400 MHz, DMSO-D6) δ ppm 1.70 (s, 6 H) 6.72 (dd, 1 H) 7.19 (tt, 1 H) 7.26 - 7.34 (m, 3 H) 7.39 (dd, 2 H) 7.45 (dd, 1 H) 7.96 (dd, 1 H) 8.28 (s, 1 H) 10.81 (s, 1 H) 12.54 (br. s., 1 H);

$[M+H]^+$ 379; HRMS ($ES^+$) calcd 379.1401, found 379.1410

**Example 119**

**N-(1-methyl-1-phenylethyl)-3-[(4-phenoxybenzoyl)amino]-1H-furo[3,2-c]pyrazole-5-carboxamide**

$^{1}$H NMR (400 MHz, DMSO-D6) δ ppm 1.70 (s, 6 H) 7.10 (d, 2 H) 7.12 - 7.16 (m, 2 H) 7.19 (tt, 1 H) 7.26 (tt, 1 H) 7.28 - 7.34 (m, 3 H) 7.37 - 7.43 (m, 2 H) 7.45 - 7.50 (m, 2 H) 8.09 (d, 2 H) 8.30 (s, 1 H) 10.85 (s, 1 H) 12.50 (br. s., 1 H); [M+H]$^{+}$ 481; LC-MS: RT 6.37

**Example 120**

**N-(1-methyl-1-phenylethyl)-3-({4-[(1-methylpiperidin-4-yl)oxy]benzoyl}amino)-1H-furo[3,2-c]pyrazole-5-carboxamide**

$^{1}$H NMR (400 MHz, DMSO-D6) δ ppm 1.62 - 1.77 (m, 8 H) 1.92 - 2.06 (m, 2 H) 2.19 - 2.42 (m, 5 H) 2.62 - 2.79 (m, 2 H) 4.48 - 4.60 (m, 1 H) 7.09 (d, 2 H) 7.19 (tt, 1 H) 7.25 - 7.34 (m, 3 H) 7.36 - 7.44 (m, 2 H) 8.02 (d, 2 H) 8.29 (s, 1 H) 10.72 (s, 1 H) 12.48 (br. s., 1 H) [M+H]$^{+}$ 502; HRMS (ES$^{+}$) calcd 502.2449, found 502.2450. LC-MS: RT 3.81

**Example 121**

**N-(1-methyl-1-phenylethyl)-3-({4-[(4-methylpiperazin-1-yl)methyl]benzoyl}amino)-1H-furo[3,2-c]pyrazole-5-carboxamide**

$^{1}$H NMR (400 MHz, DMSO-D6) δ ppm 1.70 (s, 6 H) 2.27 (s, 3 H) 2.35 - 2.50 (m, 4 H) 2.88 - 3.12 (m, 4 H) 3.57 (s, 2 H) 7.19 (tt, 1 H) 7.30 (dd, 2 H) 7.33 (s, 1 H) 7.40 (dd, 2 H) 7.47 (d, 2 H) 8.01 (d, 2 H) 8.29 (s, 1 H) 10.86 (s, 1 H) 12.51 (br. s., 1 H) [M+H]$^{+}$ 501; HRMS (ES$^{+}$) calcd 501.2609, found 501.2619; LC-MS: RT 3.56

**Example 122**

**N-(1-methyl-1-phenylethyl)-3-{[4-(morpholin-4-ylmethyl)benzoyl]amino}-1H-furo[3,2-c]pyrazole-5-carboxamide**

$^{1}$H NMR (400 MHz, DMSO-D6) δ ppm 1.68 (s, 6 H) 2.34 - 2.42 (m, 4 H) 3.55 (s, 2 H) 3.56 - 3.62 (m, 4 H) 7.18 (tt, 1 H) 7.24 - 7.34 (m, 3 H) 7.38 (dd, 2 H) 7.47 (d, 2 H) 8.00 (d, 2 H) 8.26 (s, 1 H) 10.84 (s, 1 H) 12.49 (s, 1 H); [M+H]$^{+}$ 488; HRMS (ES$^{+}$) calcd 488.2292, found 488.2291

**Example 123**

**N-(1-methyl-1-phenylethyl)-3-{[(1-methyl-1H-pyrrol-2-yl)carbonyl]amino}-1H-furo[3,2-c]pyrazole-5-carboxamide**

[M+H]$^{+}$ 392; ; LC-MS: RT 4.84

**Example 124**

**N-(1-methyl-1-phenylethyl)-3-{[4-(pyrrolidin-1-ylmethyl)benzoyl]amino}-1H-furo[3,2-c]pyrazole-5-carboxamide**

$^{1}$H NMR (400 MHz, DMSO-D6) δ ppm 1.67 (s, 6 H) 1.68 - 1.75 (m, 4 H) 2.41 - 2.47 (m, 4 H) 3.65 (s, 2 H) 7.17 (tt, 1 H) 7.24 - 7.32 (m, 3 H) 7.37 (dd, 2 H) 7.43 (d, 2 H) 7.98 (d, 2 H) 8.26 (s, 1 H) 10.82 (s, 1 H) 12.49 (br. s., 1 H); [M+H]$^{+}$ 472; HRMS (ES$^{+}$) calcd 472.2343, found 472.2328; LC-MS: RT 5.54.

**Example 125**

**N-(1-methyl-1-phenylethyl)-3-[(pyridin-2-ylcarbonyl)amino]-1H-furo[3,2-c]pyrazole-5-carboxamide**

$^{1}$H NMR (400 MHz, DMSO-D6) δ ppm 1.67 (s, 6 H) 7.17 (tt, 1 H) 7.25 - 7.31 (m, 2 H) 7.32 (s, 1 H) 7.35 - 7.42 (m, 2 H) 7.65 - 7.72 (m, 1 H) 8.04 - 8.10 (m, 1 H) 8.13 - 8.17 (m, 1 H) 8.28 (s, 1 H) 8.71 - 8.77 (m, 1 H) 10.69 (br. s., 1 H) 12.56 (br. s., 1 H); [M+H]$^{+}$ 390; HRMS (ES$^{+}$) calcd 390.1560, found 390.1560; LC-MS: RT 4.72

**Example 126**

**3-(2-furoylamino)-N-(1-phenylcyclopropyl)-1H-furo[3,2-c]pyrazole-5-carboxamide**

$^1H$ NMR (400 MHz, DMSO-D6) δ ppm 1.18 - 1.34 (m, 4 H) 6.70 (dd, 1 H) 7.16 (tt, 1 H) 7.19 - 7.23 (m, 2 H) 7.24 - 7.31 (m, 2 H) 7.33 (s, 1 H) 7.43 (dd, 1 H) 7.94 (br. d, 1 H) 9.18 (s, 1 H) 10.76 (s, 1 H) 12.53 (s, 1 H); $[M+H]^+$ 377; HRMS ($ES^+$) calcd 377.1244, found 377.1260; LC-MS: RT 4.20

**Example 127**

**3-{[(5-methylisoxazol-3-yl)carbonyl]amino}-N-(1-methyl-1-phenylethyl)-1H-furo[3,2-c]pyrazole-5-carboxamide**

$^1H$ NMR (400 MHz, DMSO-D6) δ ppm 1.68 (s, 6 H) 2.50 (s, 3 H) 6.71 (br. s., 1 H) 7.18 (tt, 1 H) 7.29 (dd, 2 H) 7.32 (s, 1 H) 7.38 (dd, 2 H) 8.29 (s, 1 H) 11.08 (s, 1 H) 12.61 (s, 1 H); $[M+H]^+$ 394; HRMS ($ES^+$) calcd 394.1510, found 394.1520; LC-MS: RT 4.61.

**Example 128**

**3-{[(1,3-dimethyl-1 H-pyrazol-5-yl)carbonyl]amino}-N-(1-methyl-1-phenylethyl)-1H-furo[3,2-c]pyrazole-5-carboxamide**

$^1H$ NMR (400 MHz, DMSO-D6) δ ppm 1.70 (s, 6 H) 2.21 (s, 3 H) 4.04 (s, 3 H) 6.95 (br. s., 1 H) 7.20 (tt, 1 H) 7.31 (dd, 2 H) 7.35 (s, 1 H) 7.40 (dd, 2 H) 8.33 (s, 1 H) 10.82 (s, 1 H) 12.54 (s, 1 H); $[M+H]^+$ 407; HRMS ($ES^+$) calcd 407.1826, found 407.1839; LC-MS: RT 4.23

**Example 129**

**N-[1-(2-fluorophenyl)-1-methylethyl]-3-(2-furoylamino)-1H-furo[3,2-c]pyrazole-5-carboxamide**

$^1H$ NMR (400 MHz, DMSO-D6) δ ppm 1.76 (s, 6 H) 7.06 - 7.18 (m, 2 H) 7.23 - 7.31 (m, 1 H) 7.33 (s, 1 H) 7.35 - 7.43 (m, 1 H) 7.46 (dd, 1 H) 7.96 (br. d, 1 H) 8.27 (br. s., 1 H) 10.79 (s, 1 H) 12.53 (s, 1 H); $[M+H]^+$ 397; HRMS ($ES^+$) calcd 397.1307, found 397.1313; LC-MS: RT 4.40

**Example 130**

**3-(2-furoylamino)-N-(1-phenylcyclopentyl)-1H-furo[3,2-c]pyrazole-5-carboxamide**

$^1H$ NMR (400 MHz, DMSO-D6) δ ppm 1.65 - 1.84 (m, 6 H) 1.95 - 2.06 (m, 2 H) 6.70 (dd, 1 H) 7.12 - 7.20 (m, 1 H) 7.23 - 7.32 (m, 3 H) 7.37 - 7.46 (m, 3 H) 7.94 (s, 1 H) 8.31 (s, 1 H) 10.74 (s, 1 H) 12.52 (s, 1 H); [M+H]+ 405; HRMS ($ES^+$) calcd 405.1557, found 405.1571; LC-MS: RT 4.88

**Example 131**

**3-[(2-methyl-3-furoyl)amino]-N-(1-methyl-1-phenylethyl)-1H-furo[3,2-c]pyrazole-5-carboxamide**

$^1H$ NMR (500 MHz, DMSO-D6) δ ppm 1.68 (s, 6 H) 2.57 (s, 3 H) 7.14 (br. s., 1 H) 7.18 (tt, 1 H) 7.29 (dd, 2 H) 7.32 (s, 1 H) 7.38 (dd, 2 H) 7.59 (d, 1 H) 8.30 (s, 1 H) 10.40 (s, 1 H) 12.47 (s, 1 H); $[M+H]^+$ 393; HRMS ($ES^+$) calcd 393.1557, found 393.1555; LC-MS: RT 5.60

**Example 132**

**N-[1-(2-methoxyphenyl)-1-methylethyl]-3-[(2-thienylcarbonyl)amino]-1H-furo[3,2-c]pyrazole-5-carboxamide**

$[M+H]^+$ 425; HRMS ($ES^+$) calcd 425.1278, found 425.1282

**Example 133**

**3-{[4-(azetidin-1-ylmethyl)benzoyl]amino}-N-(1-methyl-1-phenylethyl)-1H-furo[3,2-c]pyrazole-5-carboxamide**

$^{1}$H NMR (500 MHz, DMSO-D6) δ ppm 1.68 (s, 6 H) 1.95 - 2.05 (m, 2 H) 3.16 (t, 4 H) 3.60 (s, 2 H) 7.15 - 7.21 (m, 1 H) 7.27 - 7.31 (m, 2 H) 7.31 (s, 1 H) 7.35 - 7.45 (m, 4 H) 7.98 (d, 2 H) 8.27 (s, 1 H) 10.83 (s, 1 H) 12.50 (br. s., 1 H); $[M+H]^+$ 458; HRMS ($ES^+$) calcd 458.2187, found 458.2186; LC-MS: RT 3.25

**Example 134**

**N-(1-methyl-1-phenylethyl)-3-{[(3-methyl-2-thienyl)carbonyl]amino}-1H-furo[3,2-c]pyrazole-5-carboxamide**

$^{1}$H NMR (400 MHz, DMSO-D6) δ ppm 1.70 (s, 6 H) 2.50 (s, 3 H) 7.05 (d, 1 H) 7.19 (tt, 1 H) 7.27 - 7.34 (m, 3 H) 7.40 (dd, 2 H) 7.69 (d, 1 H) 8.32 (s, 1 H) 10.45 (s, 1 H) 12.51 (s, 1 H); $[M+H]^+$ 409; HRMS ($ES^+$) calcd 409.1329, found 409.1326

**Example 135**

**N-(1-methyl-1-phenylethyl)-3-{[(5-methyl-2-thienyl)carbonyl]amino}-1H-furo[3,2-c]pyrazole-5-carboxamide**

$^{1}$H NMR (400 MHz, DMSO-D6) δ ppm 1.70 (s, 6 H) 2.52 (s, 3 H) 6.94 (d, 1 H) 7.14 - 7.23 (m, 1 H) 7.27 - 7.34 (m, 3 H) 7.39 (dd, 2 H) 7.90 (d, 1 H) 8.29 (s, 1 H) 10.84 (s, 1 H) 12.51 (s, 1 H); $[M+H]^+$ 409; HRMS ($ES^+$) calcd 409.1329, found 409.1334

**Example 136**

**3-[(4,5-dimethyl-2-furoyl)amino]-N-(1-methyl-1-phenylethyl)-1H-furo[3,2-c]pyrazole-5-carboxamide**

$^{1}$H NMR (400 MHz, DMSO-D6) δ ppm 1.70 (s, 6 H) 1.99 (s, 3 H) 2.30 (s, 3 H) 7.19 (tt, 1 H) 7.23 (s, 1 H) 7.26 - 7.35 (m, 3 H) 7.39 (dd, 2 H) 8.23 (s, 1 H) 10.56 (s, 1 H) 12.48 (s, 1 H); $[M+H]^+$ 407; HRMS ($ES^+$) calcd 407.1714, found 407.1704

**Example 137**

**3-{[(5-chloro-2-thienyl)carbonyl]amino}-N-(1-methyl-1-phenylethyl)-1H-furo[3,2-c]pyrazole-5-carboxamide**

$^{1}$H NMR (400 MHz, DMSO-D6) δ ppm 1.70 (s, 6 H) 7.19 (tt, 1 H) 7.27 - 7.34 (m, 4 H) 7.39 (dd, 2 H) 7.99 (d, 1 H) 8.30 (s, 1 H) 11.13 (s, 1 H) 12.58 (s, 1 H); $[M+H]^+$ 429; HRMS ($ES^+$) calcd 429.0783, found 429.0785.

**Example 138**

**N-(1-methyl-1-phenylethyl)-3-{[(4-methyl-2-thienyl)carbonyl]amino}-1H-furo[3,2-c]pyrazole-5-carboxamide**

$^{1}$H NMR (400 MHz, DMSO-D6) δ ppm 1.70 (s, 6 H) 2.28 (s, 3 H) 7.19 (tt, 1 H) 7.27 - 7.34 (m, 3 H) 7.39 (dd, 2 H) 7.48 (br. s., 1 H) 7.92 (br. s., 1 H) 8.30 (s, 1 H) 10.89 (s, 1 H) 12.52 (s, 1 H) $[M+H]^+$ 409; HRMS ($ES^+$) calcd 409.1329, found 409.1343

**Example 139**

**3-{[(3,5-dimethylisoxazol-4-yl)carbonyl]amino}-N-(1-methyl-1-phenylethyl)-1H-furo[3,2-c]pyrazole-5-carboxamide**

$^{1}$H NMR (400 MHz, DMSO-D6) δ ppm 1.68 (s, 6 H) 2.34 (s, 3 H) 2.57 (s, 3 H) 7.18 (tt, 1 H) 7.29 (dd, 2 H) 7.33 (s, 1 H) 7.38 (dd, 2 H) 8.24 (s, 1 H) 10.63 (s, 1 H) 12.49 (s, 1 H); $[M+H]^+$ 408; HRMS ($ES^+$) calcd 408.1666, found 408.1660.

**Example 140**

**3-[(2,5-dimethyl-3-furoyl)amino]-N-(1-methyl-1-phenylethyl)-1H-furo[3,2-c]pyrazole-5-carboxamide**

$^1H$ NMR (400 MHz, DMSO-D6) δ ppm 1.70 (s, 6 H) 2.27 (s, 3 H) 2.50 (s, 3 H) 6.73 (br. s., 1 H) 7.16 - 7.23 (m, 1 H) 7.27 - 7.34 (m, 3 H) 7.38 - 7.45 (m, 2 H) 8.33 (s, 1 H) 10.30 (br. s., 1 H) 12.47 (br. s., 1 H); $[M+H]^+$ 407; HRMS ($ES^+$) calcd 407.1714, found 407.1713; LC-MS: RT 5.55

**Example 141**

**3-[(5-methyl-2-furoyl)amino]-N-(1-methyl-1-phenylethyl)-1H-furo[3,2-c]pyrazole-5-carboxamide**

$^1H$ NMR (400 MHz, DMSO-D6) δ ppm 1.68 (s, 6 H) 2.37 (s, 3 H) 6.32 (d, 1 H) 7.14 - 7.21 (m, 1 H) 7.25 - 7.30 (m, 2 H) 7.31 (s, 1 H) 7.33 (d, 1 H) 7.36 - 7.42 (m, 2 H) 8.22 (s, 1 H) 10.63 (br. s., 1 H) 12.49 (br. s., 1 H); $[M+H]^+$ 393; HRMS ($ES^+$) calcd 393.1557, found 393.1554

**Example 142**

**3-{[(1-methyl-1H-imidazol-4-yl)carbonyl]amino}-N-(1-methyl-1-phenylethyl)-1H-furo[3,2-c]pyrazole-5-carboxamide**

$^1H$ NMR (400 MHz, DMSO-D6) δ ppm 1.68 (s, 6 H) 3.85 (s, 3 H) 7.19 (tt, 1 H) 7.30 (dd, 2 H) 7.38 (dd, 2 H) 7.80 (s, 1 H) 8.45 - 8.61 (m, 3 H); $[M+H]^+$ 393; HRMS ($ES^+$) calcd 393.1670, found 393.1664; LC-MS: RT 8.61

**Example 143**

**3-[(3-methyl-2-furoyl)amino]-N-(1-methyl-1-phenylethyl)-1H-furo[3,2-c]pyrazole-5-carboxamide**

$^1H$ NMR (400 MHz, DMSO-D6) δ ppm 1.68 (s, 6 H) 2.34 (s, 3 H) 6.58 (br. s, 1 H) 7.14 - 7.21 (m, 1 H) 7.25 - 7.33 (m, 3 H) 7.37 (m, 2 H) 7.77 (s, 1 H) 8.33 (s, 1 H) 10.42 (br. s., 1 H) 12.49 (br. s., 1 H); $[M+H]^+$ 393; HRMS ($ES^+$) calcd 393.1557, found 393.1573

**Example 144**

**N-(1-methyl-1-phenylethyl)-3-{[5-(morpholin-4-ylmethyl)-2-furoyl]amino}-1H-furo[3,2-c]pyrazole-5-carboxamide**

$^1H$ NMR (400 MHz, DMSO-D6) δ ppm 1.68 (s, 6 H) 2.38 - 2.47 (m, 4 H) 3.54 - 3.61 (m, 4 H) 3.63 (s, 2 H) 6.43 (br. s., 1 H) 6.64 (d, 1 H) 7.14 - 7.22 (m, 1 H) 7.24 - 7.34 (m, 3 H) 7.35 - 7.43 (m, 2 H) 7.79 (s, 1 H) 7.94 (d, 1 H) 8.54 (s, 1 H) 10.47 - 10.87 (m, 1 H) 12.22 - 12.68 (m, 1 H); $[M+H]^+$ 478; HRMS ($ES^+$) calcd 478.2085, found 478.2088; LC-MS: RT 4.42

**Example 145**

**N-(1-methyl-1-phenylethyl)-3-{[(1-methyl-1H-pyrazol-5-yl)carbonyl]amino}-1H-furo[3,2-c]pyrazole-5-carboxamide**

$^1H$ NMR (400 MHz, DMSO-D6) δ ppm 1.68 (s, 6 H) 4.11 (s, 3 H) 7.15 - 7.22 (m, 2 H) 7.26 - 7.32 (m, 2 H) 7.34 (s, 1 H) 7.37 - 7.42 (m, 2 H) 7.53 (d, J=1.95 Hz, 1 H) 8.32 (s, 1 H) 10.91 (s, 1 H) 12.56 (s, 1 H); $[M+H]^+$393; HRMS ($ES^+$) calcd 393.1670, found 393.1679; LC-MS: RT 4.05

**Example 146**

**N-(1-methyl-1-phenylethyl)-3-{[(1-methyl-1H-pyrazol-3-yl)carbonyl]amino}-1H-furo[3,2-c]pyrazole-5-carboxamide**

$^1H$ NMR (400 MHz, DMSO-D6) δ ppm 1.70 (s, 6 H) 3.97 (s, 3 H) 6.84 (d, 1 H) 7.15 - 7.23 (m, 1 H) 7.26 - 7.35 (m, 3 H) 7.38 - 7.43 (m, 2 H) 7.86 (d, 1 H) 8.28 (s, 1 H) 10.24 (s, 1 H) 12.49 (s, 1 H); $[M+H]^+$ 393; HRMS ($ES^+$) calcd

393.1670, found 393.1666; LC-MS: RT 4.07

**Example 147**

**N-[1-(2-fluorophenyl)-1-methylethyl]-3-[(2-thienylcarbonyl)amino]-1H-furo[3,2-c]pyrazole-5-carboxamide**

$^1$H NMR (400 MHz, DMSO-D6) δ ppm 1.76 (s, 6 H) 7.06 - 7.18 (m, 2 H) 7.20 - 7.31 (m, 2 H) 7.33 (s, 1 H) 7.35 - 7.44 (m, 1 H) 7.90 (dd, 1 H) 8.10 (br. d., 1 H) 8.32 (s, 1 H) 10.97 (br. s., 1 H) 12.54 (br. s., 1 H); $[M+H]^+$ 413; HRMS ($ES^+$) calcd 413.1078, found 413.1071

**Example 148**

**N-[1-(2,4-difluorophenyl)-1-methylethyl]-3-[(2-thienylcarbonyl)amino]-1H-furo[3,2-c]pyrazole-5-carboxamide**

$^1$H NMR (400 MHz, DMSO-D6) δ ppm 1.72 (s, 6 H) 6.96 - 7.05 (m, 1 H) 7.05 - 7.15 (m, 1 H) 7.22 (dd, 1 H) 7.32 (s, 1 H) 7.36 - 7.48 (m, 1 H) 7.87 (dd, 1 H) 8.08 (dd, 1 H) 8.36 (s, 1 H) 10.95 (s, 1 H) 12.52 (s, 1 H); $[M+H]^+$ 431; HRMS ($ES^+$) calcd 431.0984, found 431.0994

**Example 149**

**N-(1-methyl-1-phenylethyl)-3-{[4-(piperidin-1-ylmethyl)benzoyl]amino}-1H-furo[3,2-c]pyrazole-5-carboxamide**

$^1$H NMR (500 MHz, DMSO-D6) δ ppm 1.35 - 1.46 (m, 2 H) 1.46 - 1.60 (m, 4 H) 1.68 (s, 6 H) 2.26 - 2.46 (m, 4 H) 3.51 (br. s., 2 H) 7.15 - 7.21 (m, 1 H) 7.26 - 7.47 (m, 5 H) 7.51 - 7.57 (m, 2 H) 8.03 (br. d, 2 H) 8.26 (s, 1 H) 10.85 (br. s., 1 H) 12.53 (br. s., 1 H); $[M+H]^+$ 486; HRMS ($ES^+$) calcd 486.2500, found 486.25; LC-MS: RT 3.59.

**Example 150**

**N-[1-(2-fluorophenyl)-1-methylethyl]-3-({4-[(4-methylpiperazin-1-yl)methyl]benzoyl}amino)-1H-furo[3,2-c] pyrazole-5-carboxamide**

$^1$H NMR (500 MHz, DMSO-D6) δ ppm 1.75 (s, 6 H) 2.32 (br. s., 3 H) 2.36 - 2.49 (m, 4 H) 2.58 - 2.67 (m, 4 H) 3.58 (s, 2 H) 7.07 - 7.13 (m, 1 H) 7.13 - 7.17 (m, 1 H) 7.23 - 7.31 (m, 1 H) 7.36 (s, 1 H) 7.37 - 7.42 (m, 1 H) 7.47 (d, 2 H) 8.02 (d, 2 H) 8.32 (s, 1 H) 10.86 (s, 1 H) 12.53 (s, 1 H); $[M+H]^+$ 519; HRMS ($ES^+$) calcd 519.2514, found 519.2499; LC-MS: RT 3.59

**Example 151**

**N-(1-methyl-1-phenylethyl)-3-[(1,2,3-thiadiazol-4-ylcarbonyl)amino]-1H-furo[3,2-c]pyrazole-5-carboxamide**

$^1$H NMR (400 MHz, DMSO-D6) δ ppm 1.68 (s, 6 H) 7.18 (tt, 1 H) 7.29 (dd, 2 H) 7.34 (s, 1 H) 7.38 (dd, 2 H) 8.29 (s, 1 H) 9.88 (s, 1 H) 11.32 (s, 1 H) 12.63 (s, 1 H); $[M+H]^+$ 397; HRMS ($ES^+$) calcd 397.1077, found 397.1082

**Example 152**

**3-(3-furoylamino)-N-(1-methyl-1-phenylethyl)-1H-furo[3,2-c]pyrazole-5-carboxamide**

$^1$H NMR (400 MHz, DMSO-D6) δ ppm 1.70 (s, 6 H) 7.05 (d, 1 H) 7.20 (tt, 1 H) 7.31 (dd, 2 H) 7.40 (dd, 2 H) 7.81 (dd, 1 H) 8.17 (br. s., 1 H) 8.24 (br. s, 1 H) 8.43 (s, 1 H) 10.69 (s, 1 H) 12.49 (s, 1 H); $[M+H]^+$ 379; HRMS ($ES^+$) calcd 379.1401, found 379.1396; LC-MS: RT 4.43

**Example 153** **N-(1-methyl-1-phenylethyl)-3-[(3-thienylcarbonyl)amino]-1H-furo[3,2-c]pyrazole-5- carboxamide**

$^1$H NMR (400 MHz, DMSO-D6) δ ppm 1.70 (s, 6 H) 7.16 - 7.23 (m, 1 H) 7.27 - 7.35 (m, 3 H) 7.40 (dd, 2 H) 7.67 (dd, 1 H) 7.71 (dd, 1 H) 8.29 (s, 1 H) 8.45 (br. d, 1 H) 10.77 (s, 1 H) 12.51 (s, 1 H); $[M+H]^+$ 395; HRMS ($ES^+$) calcd 395.1172, found 395.1180; LC-MS: RT 4.78

**Example 154**

**N-(1-methyl-1-phenylethyl)-3-[(1,3-thiazol-4-ylcarbonyl)amino]-1H-furo[3,2-c]pyrazole-5-carboxamide**

$^1H$ NMR (400 MHz, DMSO-D6) δ ppm 1.69 (s, 6 H) 7.19 (tt, 1 H) 7.24 - 7.35 (m, 3 H) 7.39 (dd, 2 H) 8.28 (s, 1 H) 8.56 (d, 1 H) 9.28 (d, 1 H) 10.52 (s, 1 H) 12.56 (s, 1 H); $[M+H]^+$ 396; HRMS ($ES^+$) calcd 396.1125, found 396.1112.

**Example 155**

**3-{[(1-methyl-1H-imidazol-2-yl)carbonyl]amino}-N-(1-methyl-1-phenylethyl)-1H-furo[3,2-c]pyrazole-5-carboxamide**

$^1H$ NMR (400 MHz, DMSO-D6) δ ppm 1.68 (s, 6 H) 4.00 (s, 3 H) 7.12 (br. s, 1 H) 7.14 - 7.21 (m, 1 H) 7.29 (dd, 1 H) 7.34 (s, 1 H) 7.38 (dd, 2 H) 7.47 (br. s, 1 H) 8.32 (s, 1 H) 10.41 (s, 1 H); $[M+H]^+$ 393; HRMS ($ES^+$) calcd 393.1670, found 393.1656; LC-MS: RT 4.14

**Example 156**

**N-(1-methyl-1-phenylethyl)-3-{[(1,3,5-trimethyl-1H-pyrazol-4-yl)carbonyl]amino}-1H-furo[3,2-c]pyrazole-5-carboxamide**

$^1H$ NMR (400 MHz, DMSO-D6) δ ppm 1.68 (s, 6 H) 2.18 (s, 3 H) 2.28 (s, 3 H) 3.70 (s, 3 H) 5.99 (very br. s, 1 H) 7.14 - 7.23 (m, 1 H) 7.30 (dd, 2 H) 7.38 (dd, 2 H) 7.68 (s, 1 H) 8.46 (s, 1 H); $[M+H]^+$ 421; HRMS ($ES^+$) calcd 421.1983, found 421.1966

**Example 157**

**N-[1-(2-fluorophenyl)-1-methylethyl]-3-{[4-(4-methylpiperazin-1-yl)benzoyl]amino}-1H-furo[3,2-c]pyrazole-5-carboxamide**

$^1H$ NMR (400 MHz, DMSO-D6) δ ppm 1.74 (s, 6 H) 2.22 (s, 3 H) 2.39 - 2.48 (m, 4 H) 3.22 - 3.40 (m, 4 H) 7.01 (d, 2 H) 7.04 - 7.20 (m, 2 H) 7.20 - 7.29 (m, 1 H) 7.30 (s, 1 H) 7.33 - 7.44 (m, 1 H) 7.92 (d, 2 H) 8.28 (s, 1 H) 10.52 (s, 1 H) 12.41 (s, 1 H); $[M+H]^+$ 505; HRMS ($ES^+$) calcd 505.2358, found 505.2357; LC-MS: RT 3.26.

**Example 158**

**N-(1-methyl-1-phenylethyl)-3-{[4-(2-pyrrolidin-1-ylethoxy)benzoyl]amino}-1H-furo[3,2-c]pyrazole-5-carboxamide**

$^1H$ NMR (400 MHz, DMSO-D6) δ ppm 1.70 (s, 6 H) 1.71 - 1.80 (m, 4 H) 2.56 - 2.70 (m, 4 H) 2.91 (br. s., 2 H) 4.20 (t, 2 H) 7.09 (d, 2 H) 7.19 (tt, 1 H) 7.26 - 7.35 (m, 3 H) 7.36 - 7.43 (m, 2 H) 8.04 (d, 2 H) 8.29 (s, 1 H) 10.73 (s, 1 H) 12.48 (s, 1 H); $[M+H]^+$ 502; HRMS ($ES^+$) calcd 502.2449, found 502.2448

**Example 159**

**N-[1-(2-fluorophenyl)-1-methylethyl]-3-{[4-(2-pyrrolidin-1-ylethoxy)benzoyl]amino}-1H-furo[3,2-c]pyrazole-5-carboxamide**

$^1H$ NMR (400 MHz, DMSO-D6) δ ppm 1.67 - 1.74 (m, 4 H) 1.75 (s, 6 H) 2.54 - 2.62 (m, 4 H) 2.84 (br. t., 2 H) 4.18 (t, 2 H) 7.02 - 7.11 (m, 3 H) 7.12 - 7.18 (m, 1 H) 7.22 - 7.31 (m, 1 H) 7.33 (s, 1 H) 7.35 - 7.44 (m, 1 H) 8.03 (d, 2 H) 8.31 (s, 1 H) 10.71 (s, 1 H) 12.47 (s, 1 H); $[M+H]^+$ 520; HRMS ($ES^+$) calcd 520.2355, found 520.2367; LC-MS: RT 3.57

**Example 160**

**3-({4-[(isopropylamino)methyl]benzoyl}amino)-N-(1-methyl-1-phenylethyl)-1H-furo[3,2-c]pyrazole-5-carboxamide**

$^1H$ NMR (400 MHz, DMSO-D6) δ ppm 1.14 (d, 6 H) 1.68 (s, 6 H) 2.89 - 3.06 (m, 1 H) 3.96 (br. s., 2 H) 7.14 -

7.21 (m, 1 H) 7.23 - 7.32 (m, 2 H) 7.34 (s, 1 H) 7.36 - 7.42 (m, 2 H) 7.57 (d, 2 H) 8.03 (d, 2 H) 8.26 (s, 1 H) 10.87 (s, 1 H) 12.52 (br. s., 1 H); $[M+H]^+$ 460; HRMS ($ES^+$) calcd 460.2343, found 460.2328; LC-MS: RT 3.25

**Example 161**

**3-(benzoylamino)-N-(1-methyl-1-phenylethyl)-1H-furo[3,2-c]pyrazole-5-carboxamide**

$^1$H NMR (400 MHz, DMSO-D6) δ ppm 1.70 (s, 6 H) 7.20 (tt, 1 H) 7.30 (dd, 2 H) 7.33 (s, 1 H) 7.40 (dd, 2 H) 7.55 (dd, 2 H) 7.63 (tt, 1 H) 8.03 - 8.10 (m, 2 H) 8.27 (s, 1 H) 10.89 (s, 1 H) 12.52 (s, 1 H); $[M+H]^+$ 389; HRMS ($ES^+$) calcd 389.1608, found 389.1618; LC-MS: RT 5.34

**Example 162**

**3-(benzoylamino)-N-[1-(2-fluorophenyl)-1-methylethyl]-1H-furo[3,2-c]pyrazole-5-carboxamide**

$^1$H NMR (400 MHz, DMSO-D6) δ ppm 1.76 (s, 6 H) 7.04 - 7.12 (m, 1 H) 7.12 - 7.19 (m, 1 H) 7.22 - 7.33 (m, 1 H) 7.35 (s, 1 H) 7.36 - 7.45 (m, 1 H) 7.55 (dd, 2 H) 7.62 (tt, 1 H) 8.06 (dd, 2 H) 8.30 (s, 1 H) 10.88 (s, 1 H) 12.52 (s, 1 H); $[M+H]^+$ 407; HRMS ($ES^+$) calcd 407.1514, found 407.1530; LC-MS: RT 5.38.

**Example 163**

**N-[1-(2-fluorophenyl)-1-methylethyl]-3-{[4-(pyrrolidin-1-ylmethyl)benzoyl]amino}-1H-furo[3,2-c]pyrazole-5-carboxamide**

$^1$H NMR (400 MHz, DMSO-D6) δ ppm 1.69 - 1.76 (m, 4 H) 1.76 (s, 6 H) 2.42 - 2.50 (m, 4 H) 3.67 (s, 2 H) 7.05 - 7.12 (m, 1 H) 7.12 - 7.18 (m, 1 H) 7.22 - 7.32 (m, 1 H) 7.34 (s, 1 H) 7.37 - 7.43 (m, 1 H) 7.48 (d, 2 H) 8.01 (d, 2 H) 8.31 (s, 1 H) 10.84 (s, 1 H) 12.51 (br. s., 1 H); $[M+H]^+$ 490; HRMS ($ES^+$) calcd 490.2249, found 490.2254; LC-MS: RT 3.41.

**Example 164**

**N-[1-(2-fluorophenyl)-1-methylethyl]-3-{[4-(piperidin-1-ylmethyl)benzoyl]amino}-1H-furo[3,2-c]pyrazole-5-carboxamide**

$^1$H NMR (400 MHz, DMSO-D6) δ ppm 1.35 - 1.44 (m, 2 H) 1.45 - 1.57 (m, 4 H) 1.74 (s, 6 H) 2.29 - 2.39 (m, 4 H) 3.50 (s, 2 H) 7.05 - 7.10 (m, 1 H) 7.10 - 7.17 (m, 1 H) 7.21 - 7.29 (m, 1 H) 7.33 (s, 1 H) 7.34 - 7.41 (m, 1 H) 7.44 (d, 2 H) 7.99 (d, 2 H) 8.29 (s, 1 H) 10.81 (s, 1 H) 12.49 (s, 1 H); $[M+H]^+$ 504; HRMS ($ES^+$) calcd 504.2405, found 504.2422; LC-MS: RT 3.55

**Example 165**

**N-(1-methyl-1-phenylethyl)-3-[(4-morpholin-4-ylbenzoyl)amino]-1H-furo[3,2-c]pyrazole-5-carboxamide**

$^1$H NMR (400 MHz, DMSO-D6) δ ppm 1.69 (s, 6 H) 3.24 - 3.30 (m, 4 H) 3.73 - 3.80 (m, 4 H) 7.04 (d, 2 H) 7.19 (tt, 1 H) 7.27 - 7.34 (m, 3 H) 7.39 (dd, 2 H) 7.96 (d, 2 H) 8.33 (s, 1 H) 10.60 (s, 1 H) 12.44 (s, 1 H); $[M+H]^+$474; HRMS ($ES^+$) calcd 474.2136, found 474.2118

**BioloGical Testing Example 4**

The following compounds, screened according to the methods described in the pharmacology section above, were shown to have $IC_{50}$values for Aur2 inhibition below 10 nM: 1; 2; 3; 4; 7; 10; 18; 26; 27; 28; 30; 31; 32; 33; 34; 38; 39; 40; 41; 43; 44; 45; 48; 50; 51; 53; 55; 56; 57; 58; 59; 60; 62; 63; 64; 67; 68; 69; 70; 71; 72; 73; 74; 75.

## Claims

**1.** A compound of formula (I)

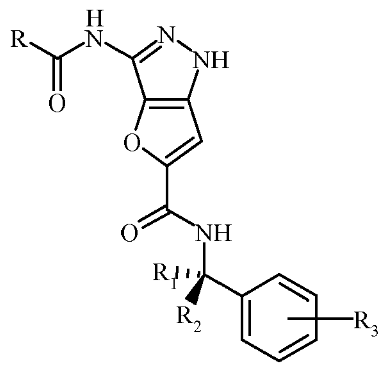

( I )

or a pharmaceutically acceptable salt of solvate thereof, wherein:

**R** is heteroaryl or phenyl group or a mono- or di-substituted heteroaryl or phenyl group in which the substituent (s) are at position(s) meta or para to the CONH linker;
**$R_1$** and **$R_2$,** which can be the same or different, is a straight or branched ($C_1$-$C_3$) alkyl or, taken together with the carbon atom to which they are bonded, **$R_1$** and **$R_2$** can form a ($C_3$-$C_6$) cycloalkyl group; and
**$R_3$** is selected from the group consisting of hydrogen, halogen, hydroxy, cyano, straight or branched ($C_1$-$C_3$) alkyl and ($C_1$-$C_3$) alkoxy.

**2.** A compound according to claim 1 selected from the group consisting of:

3-Amino-furo[3,2-c]pyrazole-1,5-dicarboxylic acid 1-ethyl ester 5-propyl ester; 3-[(Thiophene-2-carbonyl)-amino]-furo[3,2-c]pyrazole-1,5-dicarboxylic acid 1-ethyl ester 5-propyl ester;
3-(4-Fluoro-benzoylamino)-furo[3,2-c]pyrazole-1,5-dicarboxylic acid 1-ethyl ester 5-propyl ester; 3-(4-Morpholin-4-yl-benzoylamino)-furo[3,2-c]pyrazole-1,5-dicarboxylic acid 1-ethyl ester 5-propyl ester;
3-(3-Fluoro-benzoylamino)-furo[3,2-c]pyrazole-1,5-dicarboxylic acid 1-ethyl ester 5-propyl ester; 3-(4-Trifluoromethoxy-benzoylamino)-furo[3,2-c]pyrazole-1,5-dicarboxylic acid 1-ethyl ester 5-propyl ester;
3-[4-(4-Methyl-piperazin-1-ylmethyl)-benzoylamino]-furo[3,2-c]pyrazole-1,5-dicarboxylic acid 1-ethyl ester 5-propyl ester;
3-[(2-Methyl-furan-3-carbonyl)-amino]-furo[3,2-c]pyrazole-1,5-dicarboxylic acid 1-ethyl ester 5-propyl ester;
3-[(3-Methyl-thiophene-2-carbonyl)-amino]-furo[3,2-c]pyrazole-1,5-dicarboxylic acid 1-ethyl ester 5-propyl ester;
3-[(5-Methyl-thiophene-2-carbonyl)-amino]-furo[3,2-c]pyrazole-1,5-dicarboxylic acid 1-ethyl ester 5-propyl ester;
3-[(5-Chloro-thiophene-2-carbonyl)-amino]-furo[3,2-c]pyrazole-1,5-dicarboxylic acid 1-ethyl ester 5-propyl ester;
3-[(2,5-Dimethyl-furan-3-carbonyl)-amino]-furo[3,2-c]pyrazole-1,5-dicarboxylic acid 1-ethyl ester 5-propyl ester;
3-[(5-Methyl-furan-2-carbonyl)-amino]-furo[3,2-c]pyrazole-1,5-dicarboxylic acid 1-ethyl ester] 5-propyl ester;
3-[(4-Methyl-furan-2-carbonyl)-amino]-furo[3,2-c]pyrazole-1,5-dicarboxylic acid 1-ethyl ester] 5-propyl ester;
3-[(2-Methyl-2H-pyrazole-3-carbonyl)-amino]-furo[3,2-c]pyrazole-1,5-dicarboxylic acid 1-ethyl ester 5-propyl ester;
3-[(Thiophene-3-carbonyl)-amino]-furo[3,2-c]pyrazole-1,5-dicarboxylic acid 1-ethyl ester 5-propyl ester;
3-[(Thiazole-4-carbonyl)-amino]-furo[3,2-c]pyrazole-1,5-dicarboxylic acid 1-ethyl ester 5-propyl ester;
3-[(1-Methyl-1H-imidazole-2-carbonyl)-amino]-furo[3,2-c]pyrazole-1,5-dicarboxylic acid 1-ethyl ester 5-propyl ester;
3-[(1-Methyl-1H-imidazole-4-carbonyl)-amino]-furo[3,2-c]pyrazole-1,5-dicarboxylic acid 1-ethyl ester 5-propyl ester;
3-[(5-Morpholin-4-ylmethyl-furan-2-carbonyl)-amino]-furo[3,2-c]pyrazole-1,5-dicarboxylic acid 1-ethyl ester 5-propyl ester;
3-Benzoylamino-furo[3,2-c]pyrazole-1,5-dicarboxylic acid 1-ethyl ester 5-propyl ester; 3-[4-(2-Pyrrolidin-1-yl-ethoxy)-benzoylamino]-furo[3,2-c]pyrazole-1,5-dicarboxylic acid 1-ethyl ester 5-propyl ester;
3-(4-Fluoro-benzoylamino)-1H-furo[3,2-c]pyrazole-5-carboxylic acid;
3-[(4-Bromo-thiophene-2-carbonyl)-amino]-1H-furo[3,2-c]pyrazole-5-carboxylic acid;

3-[4-(4-Methyl-piperazin-1-yl)-benzoylamino]-1H-furo[3,2-c]pyrazole-5-carboxylic acid;
3-(2-Fluoro-benzoylamino)-1H-furo[3,2-c]pyrazole-5-carboxylic acid;
3-(4-Trifluoromethoxy-benzoylamino)-1H-furo[3,2-c]pyrazole-5-carboxylic acid;
3-(4-Methoxy-benzoylamino)-1H-furo[3,2-c]pyrazole-5-carboxylic acid;
3-(3-Methoxy-benzoylamino)-1H-furo[3,2-c]pyrazole-5-carboxylic acid;
3-(2-Methoxy-benzoylamino)-1H-furo[3,2-c]pyrazole-5-carboxylic acid;
3-(4-Cyano-benzoylamino)-1H-furo[3,2-c]pyrazole-5-carboxylic acid;
3-[(Furan-2-carbonyl)-amino]-1H-furo[3,2-c]pyrazole-5-carboxylic acid;
3-[4-(1-Methyl-piperidin-4-yloxy)-benzoylamino]-1H-furo[3,2-c]pyrazole-5-carboxylic acid;
3-[4-(4-Methyl-piperazin-1-ylmethyl)-benzoylamino]-1H-furo[3,2-c]pyrazole-5-carboxylic acid;
3-(4-Piperidin-1-ylmethyl-benzoylamino)-1H-furo[3,2-c]pyrazole-5-carboxylic acid;
3-(4-Azetidin-1-ylmethyl-benzoylamino)-1H-furo[3,2-c]pyrazole-5-carboxylic acid;
3-[(1-Methyl-1H-pyrrole-2-carbonyl)-amino]-1H-furo[3,2-c]pyrazole-5-carboxylic acid;
3-[(5-Methyl-isoxazole-3-carbonyl)-amino]-1H-furo[3,2-c]pyrazole-5-carboxylic acid;
3-[(2,5-Dimethyl-2H-pyrazole-3-carbonyl)-amino]-1H-furo[3,2-c]pyrazole-5-carboxylic acid;
3-[(2-Methyl-furan-3-carbonyl)-amino]-1H-furo[3,2-c]pyrazole-5-carboxylic acid;
3-[(3-Methyl-thiophene-2-carbonyl)-amino]-1H-furo[3,2-c]pyrazole-5-carboxylic acid;
3-[(5-Methyl-thiophene-2-carbonyl)-amino]-1H-furo[3,2-c]pyrazole-5-carboxylic acid;
3-[(4,5-Dimethyl-furan-2-carbonyl)-amino]-1H-furo[3,2-c]pyrazole-5-carboxylic acid;
3-[(5-Chloro-thiophene-2-carbonyl)-amino]-1H-furo[3,2-c]pyrazole-5-carboxylic acid;
3-[(Thiophene-2-carbonyl)-amino]-1H-furo[3,2-c]pyrazole-5-carboxylicacid (1-methyl-1-phenylethyl)-amide;
3-(4-Morpholin-4-ylmethyl-benzoylamino)-1H-furo[3,2-c]pyrazole-5-carboxylic acid[1-(2-fluorophenyl)-1-methyl-ethyl]-amide; and
3-[4-(4-Methyl-piperazin-1-yl)-benzoylamino]-1H-furo[3,2-c]pyrazole-5-carboxylic acid (1-phenylcyclopropyl)-amide

**3.** A a process for preparing the compounds of formula (I), and the pharmaceutically acceptable salts or solvates thereof, which process comprises:

a) reacting a bi-cyclic compound of formula (II)

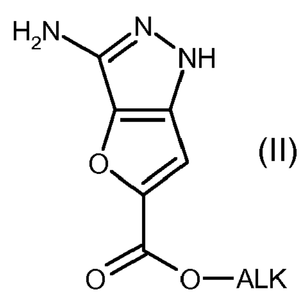

(II)

wherein ALK is a $C_1$-$C_4$ alkyl group, with a suitable pyrazole nitrogen atom protecting agent, such as ethyl chloroformate,
b) acylating the resultant compound of formula (III)

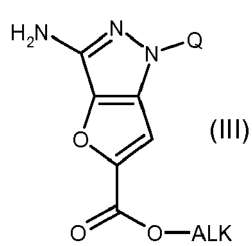

(III)

wherein ALK is as defined above and Q represents any suitable pyrazole protecting group, such as, for example, ethyloxycarbonyl, with a compound of formula (IV)

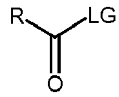
(IV)

wherein R is as defined in formula (I), and LG represents a suitable leaving group;
c) hydrolysing the alkyl ester group and removing the protective group Q from the compound of formula (V)

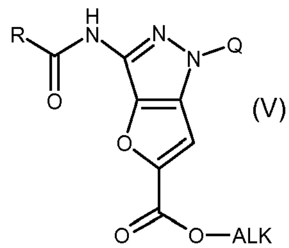
(V)

wherein ALK, R and Q are as defined above; and
d) reacting the resultant compound of formula (VI)

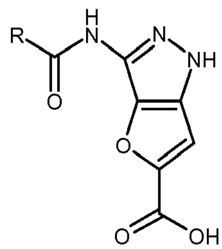
(VI)

wherein R is as defined above, with a compound of formula (VII)

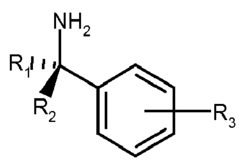
(VII)

wherein $R_1$, $R_2$ and $R_3$ are as defined in Formula (I), in the presence of a suitable condensing agent, as to obtain the compound of formula (I) and, whenever desired, converting the compound of formula (I) into a pharmaceutically acceptable salt or converting the salt thereof into the free compound of formula (I).

**4.** A pharmaceutical composition comprising a therapeutically effective amount of the compound of formula (I) or a pharmaceutically acceptable salt thereof, as defined in claim 1, and at least one pharmaceutically acceptable excipient, carrier and/or diluent.

**5.** A pharmaceutical composition according to claim 4 further comprising one or more chemotherapeutic agents.

**6.** A compound of formula (I) or a pharmaceutically acceptable salt thereof, as defined in claim 1, for use as a medicament.

**7.** Use of a compound of formula (I) or a pharmaceutically acceptable salt thereof, as defined in claim 1, in the manufacture of a medicament for treating a disorder or condition caused by or associated with an altered Aurora kinase activity.

8. Use of a compound of formula (I) or a pharmaceutically acceptable salt thereof, as defined in claim 1, in the manufacture of a medicament with antitumor activity.

9. A compound of formula (I) or a pharmaceutically acceptable salt thereof, as defined in claim 1, for use in a method for treating a disease caused by or associated with an altered Aurora kinase activity.

Europäisches Patentamt
European Patent Office
Office européen des brevets

# EUROPEAN SEARCH REPORT

Application Number
EP 08 16 7597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2006/160874 A1 (TONANI ROBERTO [IT] ET AL) 20 July 2006 (2006-07-20) * page 2, column 1, lines 12-17,54-56 * * example 18 * ----- | 1-9 | INV. C07D491/04 |
| P,A | WO 2007/138017 A (NERVIANO MEDICAL SCIENCES S R [IT]; FANCELLI DANIELE [IT]; PULICI MAUR) 6 December 2007 (2007-12-06) * the whole document * ----- | 1-9 | |
| | | | TECHNICAL FIELDS SEARCHED (IPC) |
| | | | C07D |

The present search report has been drawn up for all claims

| Place of search | Date of completion of the search | Examiner |
|---|---|---|
| The Hague | 10 December 2008 | Hacking, Michiel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 08 16 7597

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2008

| Patent document cited in search report | Publication date | Patent family member(s) | Publication date |
|---|---|---|---|
| US 2006160874 A1 | 20-07-2006 | NONE | |
| WO 2007138017 A | 06-12-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04007504 A


### Non-patent literature cited in the description


- *Current Opinion in Chemical Biology,* 1999, vol. 3, 459-465
- *Cancer Res.,* 1999, vol. 59 (9), 2041-4
- *J. Natl. Cancer Inst.,* 2002, vol. 94 (17), 1320-9
- *Molecular Cancer Therapeutics,* 2003, vol. 2, 589-595
- **T. HIGUCHI ; V. STELLA.** Pro-drugs as Novel Delivery Systems. *A.C.S. Symposium Series,* 1987, vol. 14
- Bioreversible Carriers in Drug Design. American Pharmaceutical Association and Pergamon Press, 1987